# EUROPEAN PATENT APPLICATION

(11) **EP 4 332 091 A1**
(43) Date of publication of application: **06.03.2024**
(21) Application number: 22794686.0
(22) Date of filing: 19.04.2022
(51) Int. Cl.: C07D 207/34, C07D 231/14, C07B 43/06, A61K 31/40, A61P 9/12

(54) **PREPARATION METHOD FOR PYRROLE AMIDE COMPOUND**

(30) Priority: 26.04.2021 CN 202110452047
(71) Applicant: Sunshine Lake Pharma Co., Ltd., Dongguan, Guangdong 523000 (CN)
(72) Inventor: WANG, Jiancheng, Dongguan, Guangdong 523871 (CN); ZHANG, Yingxun, Dongguan, Guangdong 523871 (CN); YANG, Chuanwen, Dongguan, Guangdong 523871 (CN); ZONG, Qiao, Dongguan, Guangdong 523871 (CN); MA, Facheng, Dongguan, Guangdong 523871 (CN); DING, Xiaohong, Dongguan, Guangdong 523871 (CN); WANG, Xiaojun, Dongguan, Guangdong 523871 (CN)
(74) Representative: Hoffmann Eitle
(86) International application number: PCT/CN2022/087723
(87) International publication number: WO 2022/228215

(57) **Abstract**

A preparation method for a pyrrole amide compound, as shown in formula (II). The preparation method comprises: reacting a compound of formula (III) with a compound of formula (IV) in the presence of a palladium catalyst and a ligand, wherein X is Br or I; R is H, D, F, Cl, Br, C₁₋₄ alkyl, C₁₋₄ alkoxy, C₁₋₄ haloalkyl or C₁₋₄ haloalkoxy; and PG₁ is benzyl or substituted benzyl.

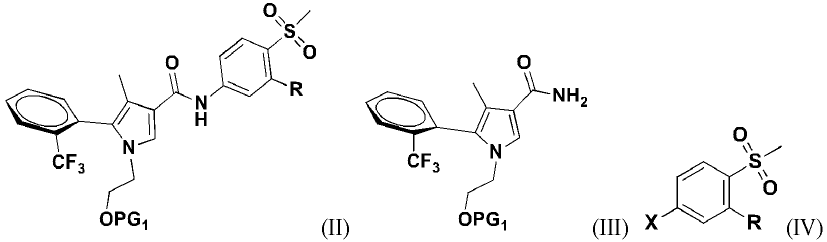

## Description

### FIELD OF THE INVENTION

The invention relates to the field of medicinal chemistry, in particular to a preparation method for pyrrole amide compound, and also to important intermediate compounds and preparation methods thereof.

### BACKGROUND ART

International application WO 2006012642 A2 discloses a pyrrole derivative for modulating the activity of one or more steroid nuclear receptors - a racemic compound of Esaxerenone (CS-3150) and a preparation method thereof; the structural formula of the compound is as follows:

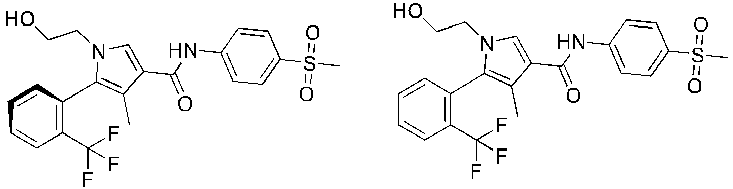

Esaxerenone , racemic compound of Esaxerenone.

International application WO 2008126831 A1 discloses Esaxerenone, which can be used to treat diseases such as hypertension; the application also discloses a preparation method thereof, and the specific process is as follows; the method uses chiral column resolution to obtain the target compound with a single stereo configuration:

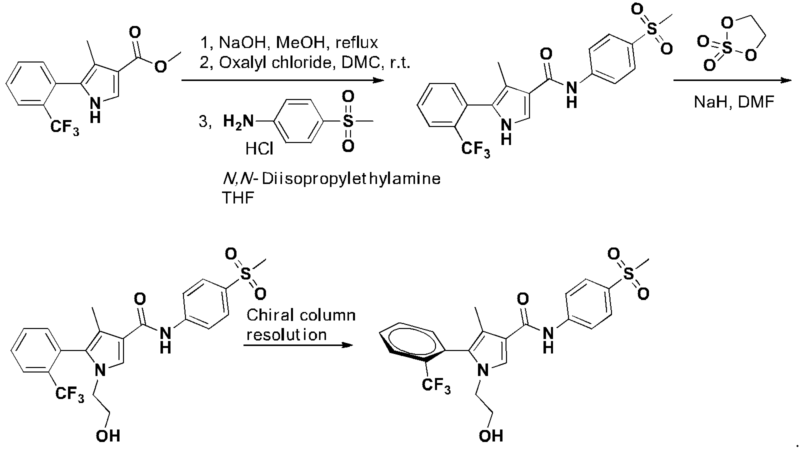

International application WO 2015030010 A1 discloses an intermediate for the preparation of Esaxerenone compound and preparation methods thereof, wherein the methods use enzymatic resolution or optically active amine resolution to prepare an intermediate compound with S configuration, which is then reacted with 4-(methylsulfonyl)aniline in the presence of ethylmagnesium bromide to obtain the target compound; and the said three methods are as follows. The methods of this patent application use expensive Grignard reagent, and the Grignard reagent is used in a large amount and is added dropwise at relatively high temperature, which has potential safety hazards and is not conducive to industrial production. In addition, in the method two, sodium borohydride and boron trifluoride-ether complex are used to reduce the carboxyl group, the reaction conditions are dangerous, and it is not easy to scale up production.

### Method one:

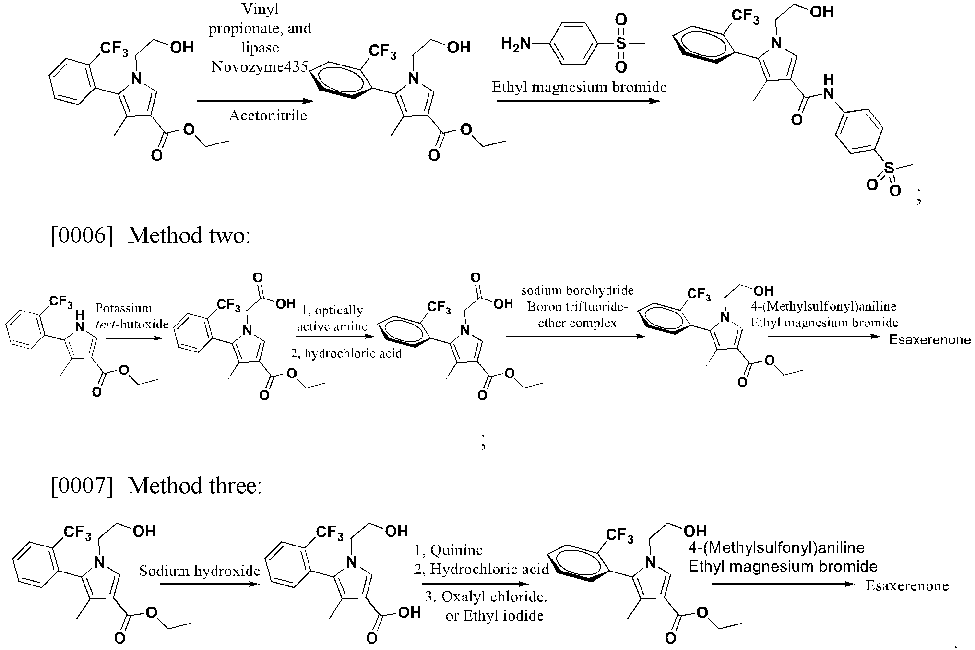

International application WO 2014168103 A1 discloses another method for preparing Esaxerenone as shown below. In this method, carboxylic acid and acyl chloride intermediate compounds with S configuration are sequentially prepared, and then reacted with 4-(methylsulfonyl)aniline to obtain the target product; the acyl chloride intermediate compound is unstable and easily deteriorated, which is not conducive to the control of subsequent reactions and product quality, and is also not conducive to industrial production.

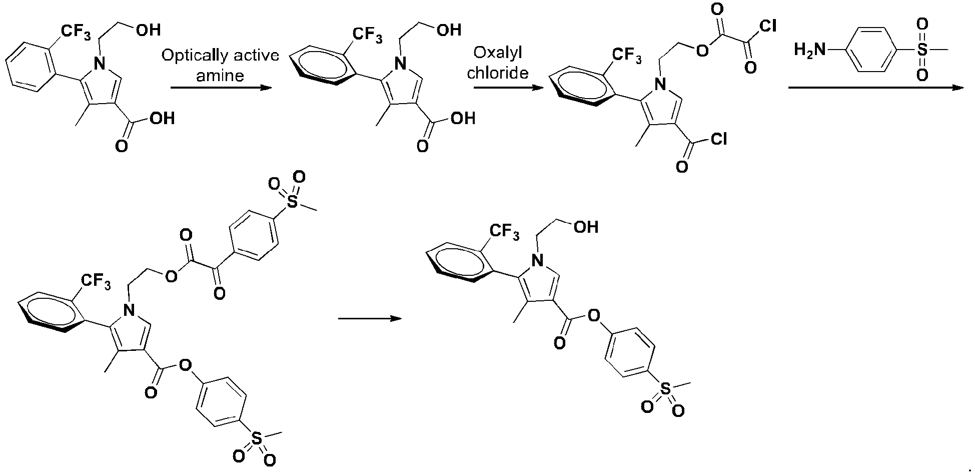

### SUMMARY

The present invention provides new methods for preparing pyrrole amide compounds, wherein the pyrrole amide compounds include Esaxerenone and its analogues. At the same time, the present invention also relates to important intermediates in the methods and preparation methods thereof. The preparation methods of the invention have mild conditions, simple operation, safety and controllability, and high yield, and are suitable for industrial production.

In one aspect, the present invention provides a method for preparing compound of formula (IIA), 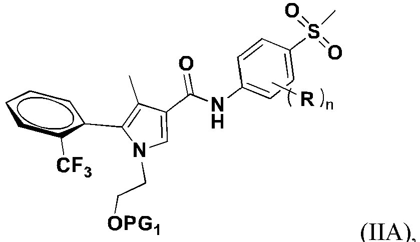 wherein, n is 0, 1, 2, 3 or 4, and R and PG₁ have the meanings described in the present invention. Specifically, the preparation method is as described in the present invention.

In some embodiments, the present invention provides a method for preparing compound of formula (II): 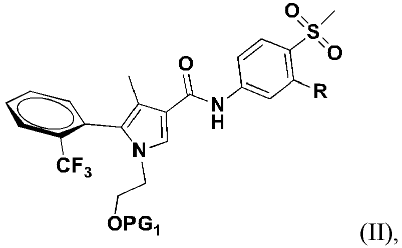
wherein, the method comprises: reacting a compound of formula (III) with a compound of formula (IV) to obtain a compound of formula (II); 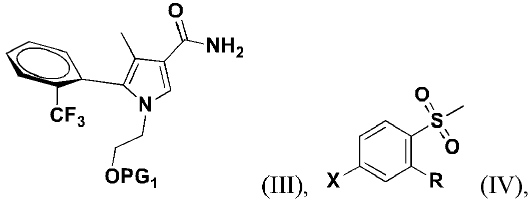
wherein X is Br or I;
R is H, D, F, Cl, Br, C₁₋₄ alkyl, C₁₋₄ alkoxy, C₁₋₄ haloalkyl or C₁₋₄ haloalkoxy; and
PG₁ is a suitable hydroxyl protecting group, including but not limited to, alkyl, alkenyl, alkoxyalkyl, alkylthioalkyl, silylalkyl, benzyl or substituted benzyl, and the like. Preferably, PG₁ is benzyl or substituted benzyl. More preferably, PG₁ is benzyl, p-methylbenzyl orp-methoxybenzyl.

In other embodiments, the reaction is carried out in the presence of base 1. Preferably, the base 1 is potassium carbonate, cesium carbonate, sodium carbonate, potassium *tert*-butoxide or potassium phosphate. More preferably, the base 1 is potassium carbonate, cesium carbonate, potassium *tert*-butoxide or potassium phosphate.

In still other embodiments, the amount of the base 1 is multiple times the molar amount of the compound of formula (III), including but not limited to 1.2 to 6.0 times. In some other embodiments, the amount of the base 1 is 1.5 to 3.0 times the molar amount of the compound of formula (III), preferably 1.5 to 2.0 times.

In other embodiments, the reaction is carried out in the presence of a catalyst, which is a metal catalyst.

In yet other embodiments, the catalyst described herein is a palladium catalyst. Preferably, the palladium catalyst is Pd₂(dba)₃, Pd(dppf)Cl₂·CH₂Cl₂ or Pd(OAc)₂.

In yet other embodiments, the amount of the catalyst herein is 0.5%~5% of the molar amount of the compound of formula (III), preferably 1%~3%, more preferably 1.5%~ 3%.

In other embodiments, the reaction described herein is carried out in the presence of a ligand. In yet other embodiments, the ligand is DMEDA, Xantphos, X-phos or S-phos. In yet other embodiments, the ligand is Xantphos, X-phos or S-phos.

In some other embodiments, the amount of the ligand herein is 0.5%~5% of the molar amount of the compound of formula (III), preferably 1%~3%, more preferably 1.5% ~3%.

In other embodiments, the reaction described herein is carried out in the presence of a catalyst and a ligand. In some embodiments, the molar ratio of catalyst to ligand is 1:1. In some other embodiments, the catalyst is Pd(OAc)₂, and the ligand is Xantphos, S-phos or X-phos; or the catalyst is Pd₂(dba)₃, and the ligand is Xantphos, S-phos or X-phos; or the catalyst is Pd(dppf)Cl₂·CH₂Cl₂, and the ligand is Xantphos, S-phos or X-phos; or the catalyst is Pd(dppf)Cl₂·CH₂Cl₂, and the ligand is Xantphos.

In yet other embodiments, the catalyst is CuI, the ligand is DMEDA, and the base 1 is potassium carbonate.

In other embodiments, the reaction is carried out in solvent 1, and the solvent 1 is toluene, dioxane, *tert*-butanol (*t*-BuOH), *tert*-amyl alcohol (*t*-AmOH), dimethyl ether (DME), cyclopentyl methyl ether (CPME), *N*,*N*-dimethylacetamide (DMAC), *N*,*N*-dimethylformamide (DMF), water or any combinations thereof.

In other embodiments, the reaction temperature of the reaction is 45°C to 110°C, preferably 45°C to 100°C or 55°C to 110°C, more preferably 55°C to 100°C or 58°C to 100°C, further preferably 55°C to 83°C or 58°C to 83°C.

In other embodiments, the crude product of the compound of formula (II) prepared by the method described herein can be purified by a purification method. The purification method includes, but is not limited to, recrystallization, removing impurities by palladium-removing silica gel and/or activated carbon, etc.; and recrystallization can be performed multiple times if necessary. The recrystallization can be carried out by dissolving the crude product in a good solvent, the mixture is stirred and dissolved, then a poor solvent is added, or a solution of the crude product is added to a poor solvent, and the mixture is stirred to precipitate a solid. The dissolving process can be carried out at normal temperature or under the condition of heating and increasing temperature. Preferably, after dissolving the crude product in a good solvent, impurities can be removed by means of palladium-removing silica gel and/or activated carbon, and then the solution of the crude product is added to a poor solvent to precipitate a solid. In some embodiments, the good solvent is ethanol, and the poor solvent is water; preferably, the total volume ratio of ethanol to water may be from about 1:1.5 to about 1:15, more preferably from about 1:1.8 to about 1:15.

In other embodiments, the preparation method of the compound of formula (II) described herein also includes the preparation method of the compound of formula (III), which comprises the following steps:

Step c: the compound of formula (VI) reacts with thionyl chloride, oxalyl chloride or *N*,*N*'-carbonyldiimidazole (CDI) to obtain the compound of formula (V), 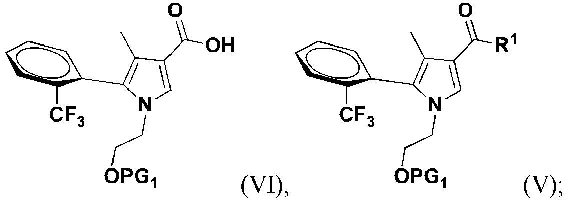
Step d: the compound of formula (V) undergoes an ammonolysis reaction to obtain the compound of formula (III);
wherein, R¹ is Cl or 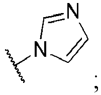 and PG₁ has the meaning described in the present invention.

In some other embodiments, the reaction of step c is carried out at 0°C to room temperature.

In yet other embodiments, the compound of formula (V) is a compound of formula (Va) or (Vb),

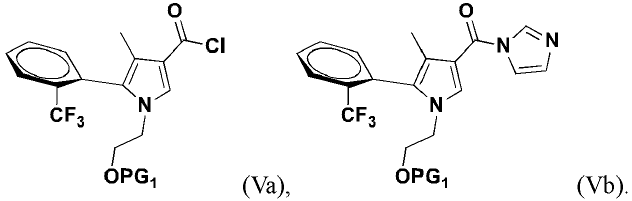

In some other embodiments, in step c, the compound of formula (VI) is reacted with thionyl chloride or oxalyl chloride to obtain the compound of formula (Va), 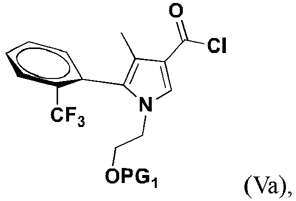 wherein, the reaction is carried out in a suitable solvent.

In still other embodiments, the suitable solvent includes, but is not limited to, dichloromethane (DCM) or tetrahydrofuran (THF), and the like.

In some other embodiments, in step c, the compound of formula (VI) is reacted with *N*,*N'*-carbonyldiimidazole (CDI) to obtain the compound of formula (Vb), 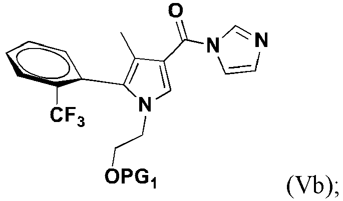 wherein, the reaction is carried out in a solvent such as *N*,*N*-dimethylformamide (DMF) or DMAC at room temperature.

In still other embodiments, the ammonolysis reaction in step d is carried out in the presence of an amination reagent. Preferably, the amination reagent is ammonium hydroxide or an ammonium salt reagent; preferably, the amination reagent is ammonium hydroxide, ammonium bromide or NH₄SCN.

In some other embodiments, the ammonolysis reaction in step d is further carried out in the presence of base 2, and the base 2 is potassium carbonate, sodium carbonate or cesium carbonate.

In yet other embodiments, the amination reagent in step d is ammonium bromide or NH₄SCN; and the ammonolysis reaction in step d is carried out in the presence of base 2, which is potassium carbonate, sodium carbonate or cesium carbonate.

In some other embodiments, the temperature of the ammonolysis reaction in step d is from 0°C to room temperature (eg, from 10°C to 40°C). Optionally, the temperature of the ammonolysis reaction in step d is 0°C. Optionally, the temperature of the ammonolysis reaction in step d is room temperature.

In some other embodiments, the crude product of the compound of formula (III) obtained by the ammonolysis reaction of step d can be further purified by slurrying or recrystallization.

In some other embodiments, the recrystallization includes: adding the crude product of the compound of formula (III) to solvent a, heating and stirring until the system is dissolved, then adding solvent b, cooling, and stirring to precipitate a solid.

In some other embodiments, in the recrystallization, after adding the crude product of the compound of formula (III) to solvent a, the mixture was heated to about 80°C, and stirred until the system is dissolved.

In some other embodiments, the solvent a is isopropyl acetate or *tert*-butyl acetate, and the solvent b is *n*-hexane, *n*-heptane or cyclohexane.

In still other embodiments, the volume ratio of solvent a to solvent b is 1:2 to 4:3.

In some other embodiments, the solvent a is isopropyl acetate, and the solvent b is cyclohexane, wherein the volume ratio of cyclohexane to isopropyl acetate is 1: 1 to 2:1.

In some other embodiments, the solvent a is *tert*-butyl acetate, and the solvent b is cyclohexane, wherein the volume ratio of cyclohexane to *tert*-butyl acetate is 1: 1 to 3:4, preferably 3:4.

In some other embodiments, the total volume of solvent a and solvent b is 6 to 7 times the mass of the crude product of the compound of formula (III); that is, based on the mass of the crude product of the compound of formula (III), the total volume of solvent a and solvent b is 6 mL/g to 7 mL/g.

In some other embodiments, the preparation method of the compound of formula (III) described herein also includes the preparation method of the compound of formula (VI), which comprises the following steps:
step a: a compound of formula (VIII) is reacted with benzyl bromide or substituted benzyl bromide to obtain a compound of formula (VII); 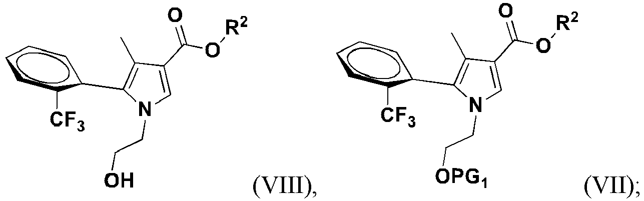
step b: the compound of formula (VII) is hydrolyzed to obtain the compound of formula (VI);
wherein, R² is C₁₋₄ alkyl, benzyl or substituted benzyl; and
PG₁ is benzyl or substituted benzyl.

In some embodiments, the R² is methyl, ethyl, *n*-propyl, isopropyl, benzyl or substituted benzyl.

In some embodiments, the molar amount of benzyl bromide or substituted benzyl bromide in step a is 1.0 time or more than 1.0 time that of the compound of formula (VIII); preferably, the molar amount of benzyl bromide or substituted benzyl bromide is 1.0 to 4.0 times that of the compound of formula (VIII); preferably, the molar amount of benzyl bromide or substituted benzyl bromide is 1.0 to 2.5 times that of the compound of formula (VIII); preferably, the molar amount of benzyl bromide or substituted benzyl bromide is 1.1 to 1.7 times that of the compound of formula (VIII); more preferably, the molar amount of benzyl bromide or substituted benzyl bromide is 1.5 to 1.7 times that of the compound of formula (VIII).

In some embodiments, the reaction of step a is carried out in the presence of base a, and the base a is sodium *tert*-pentoxide, sodium *tert*-butoxide, sodium ethoxide, sodium methoxide, sodium hydroxide, tetrabutylammonium hydroxide, potassium *tert*-pentoxide, potassium *tert*-butoxide or potassium hydroxide; preferably, the reaction of step a is carried out in the presence of sodium *tert*-pentoxide, sodium ethoxide, sodium methoxide, sodium hydroxide, tetrabutylammonium hydroxide or potassium hydroxide; more preferably, the reaction of step a is carried out in the presence of sodium *tert*-pentoxide, sodium ethoxide or potassium hydroxide; further preferably, the reaction of step a is carried out in the presence of sodium *tert*-pentoxide.

In other embodiments, the molar amount of the base a is 1.0 time or more than 1.0 time that of the compound of formula (VIII), preferably 1.0 to 4.0 times, more preferably 1.1 to 2.5 times.

In some other embodiments, the reaction of step a is carried out in the presence of sodium *tert*-pentoxide, and the molar amount of sodium *tert*-pentoxide is 1.0 time or more than 1.0 time that of the compound of formula (VIII); preferably, the molar amount of sodium *tert*-pentoxide is 1.0 to 4.0 times that of the compound of formula (VIII); preferably, the molar amount of sodium *tert*-pentoxide is 1.0 to 2.5 times that of the compound of formula (VIII); preferably, the molar amount of sodium *tert*-pentoxide is 1.1 to 1.7 times that of the compound of formula (VIII); more preferably, the molar amount of sodium *tert*-pentoxide is 1.5 to 1.7 times that of the compound of formula (VIII).

In some embodiments, the reaction of step a can be further carried out in the presence of sodium iodide. Preferably, the reaction of step a is carried out in the presence of a catalytic amount of sodium iodide; the catalytic amount includes but is not limited to 0.1, 0.15 or 0.2 equivalents.

In some other embodiments, the base a can be added at normal temperature (i.e., room temperature) or at low temperature or at low temperature to normal temperature.

In some other embodiments, the reaction of step a is carried out in the presence of sodium *tert*-pentoxide, which is added at normal temperature or at low temperature or at low temperature to normal temperature; preferably, the sodium *tert*-pentoxide is added at 10°C to 30°C; more preferably, the sodium *tert*-pentoxide is added at 10°C.

In some embodiments, the reaction temperature of step a is from room temperature to 90°C, preferably from room temperature to 80°C, or, preferably from 30°C to 90°C; further preferably, the reaction temperature of step a is 30°C to 90°C, more preferably from 30°C to 60°C.

In some embodiments, the reaction of step a can be carried out under N₂ or without N₂; preferably, the reaction of step a is carried out under N₂.

In some embodiments, the reaction solvent in step a is acetonitrile, tetrahydrofuran (THF), *N*-methylpyrrolidone (NMP), dimethylsulfoxide (DMSO), *N*,*N*-dimethylacetamide (DMAC) or *N*,*N*-dimethylformamide (DMF); preferably, the reaction solvent in step a is acetonitrile, *N*,*N*-dimethylacetamide or *N*,*N*-dimethylformamide; more preferably, the reaction solvent in step a is *N*,*N*-dimethylacetamide or *N*,*N*-dimethylformamide.

In some embodiments, in step a, based on the mass of the compound of formula (VIII), the amount of the reaction solvent is 5 mL/g to 50 mL/g, preferably 10 mL/g to 20 mL/g.

In some embodiments, the reaction of step b is carried out in the presence of base b; wherein the base b is sodium hydroxide or potassium hydroxide, etc.; preferably, the base b is potassium hydroxide.

In some embodiments, the reaction of step b is carried out in alcoholic solvent. The alcoholic solvent is preferably methanol or ethanol.

In another aspect, the present invention provides a method for preparing a pyrrole amide compound of formula (IA), 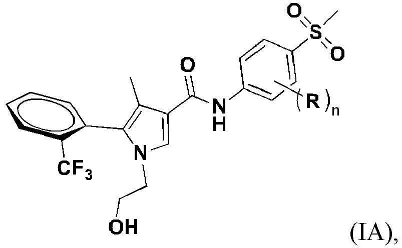 wherein, n is 0, 1, 2, 3 or 4, and R has the meaning described in the present invention. Specifically, the preparation method is as described in the present invention.

In one aspect, the present invention provides a method for preparing a compound of formula (I), 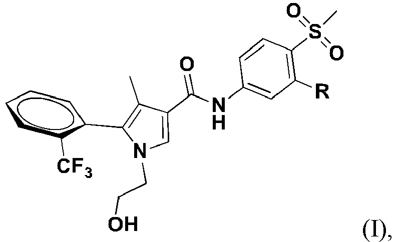
wherein, R is H, D, F, Cl, Br, C₁₋₄ alkyl, C₁₋₄ alkoxy, C₁₋₄ haloalkyl or C₁₋₄ haloalkoxy;
wherein, the method comprises the following steps: a compound of formula (II) undergoes a deprotection reaction to obtain the compound of formula (I), 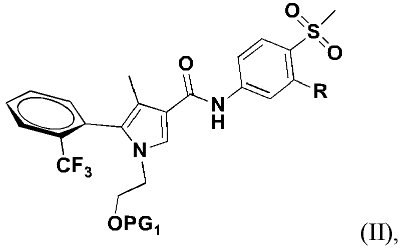
wherein, PG₁ is a suitable hydroxyl protecting group, including but not limited to, alkyl, alkenyl, alkoxyalkyl, alkylthioalkyl, silylalkyl, benzyl or substituted benzyl, etc.; preferably, PG₁ is benzyl or substituted benzyl; more preferably, PG₁ is benzyl, *p*-methylbenzyl or *p*-methoxybenzyl.

In some embodiments, the deprotection reaction is carried out in the presence of palladium on carbon under H₂.

In some embodiments, the deprotection reaction can be carried out under an acidic condition or in the presence of an acidic substance.

In some embodiments, the deprotection reaction is carried out in the presence of a Lewis acid. In other embodiments, the Lewis acid is zinc chloride, aluminum chloride, titanium tetrachloride, or the like. In other embodiments, the Lewis acid is zinc chloride, aluminum chloride, or the like. In other embodiments, the Lewis acid is aluminum chloride or titanium tetrachloride.

In some other embodiments, the acidic condition refers to the condition under which an acid is present, and the acid is a suitable acid appropriate for the deprotection reaction, including but not limited to hydrochloric acid, acetic acid, trifluoroacetic acid, sulfuric acid, or the like. In yet other embodiments, the acid is hydrochloric acid, acetic acid, sulfuric acid, trifluoroacetic acid or solutions thereof. In yet other embodiments, the acid is hydrochloric acid, acetic acid, trifluoroacetic acid or solutions thereof. Preferably, the acid is hydrochloric acid, sulfuric acid, trifluoroacetic acid or solutions thereof.

In some embodiments, the acidic condition is in the presence of zinc chloride, aluminum chloride, titanium tetrachloride, hydrochloric acid, sulfuric acid, acetic acid or trifluoroacetic acid.

In other embodiments, the acidic condition is in the presence of zinc chloride, hydrochloric acid, acetic acid or trifluoroacetic acid.

In some other embodiments, based on the mass of the compound of formula (II), the amount of acid is 0.17 mL/g to 0.34 mL/g.

In still other embodiments, the acid is hydrochloric acid, preferably, based on the mass of the compound of formula (II), the amount of hydrochloric acid is 0.17 mL/g to 0.34 mL/g.

In still other embodiments, the hydrochloric acid refers to an aqueous solution of hydrogen chloride. Specifically, the concentration of the hydrochloric acid is greater than 1 mol/L; preferably, the concentration of the hydrochloric acid is 1 mol/L to 12 mol/L; preferably, the concentration of the hydrochloric acid is 3 mol/L to 12 mol /L.

In some embodiments, the reaction temperature of the deprotection reaction is 50°C to 75°C; preferably, the reaction temperature is 55°C to 70°C.

In some embodiments, the deprotection reaction of the present invention can be carried out under atmospheric pressure (0.1 MPa), or under a condition higher than atmospheric pressure. In other embodiments, the pressure of the deprotection reaction of the present invention is 0.1 MPa to 3.8 MPa. In still other embodiments, the pressure of the deprotection reaction of the present invention is 0.8 MPa to 3.8 MPa.

In some embodiments, the deprotection reaction of the present invention is carried out in an alcoholic solvent, and the alcoholic solvent is methanol, ethanol, isopropanol, *tert*-butanol or *tert*-amyl alcohol. Preferably, based on the mass of the compound of formula (II), the amount of the alcoholic solvent can be 10 mL/g.

In some embodiments, the crude product obtained by the deprotection reaction of the present invention can be purified by recrystallization to obtain the pure compound of formula (I).

In other embodiments, the recrystallization herein is carried out in a mixed solvent of ethanol and water. In still other embodiments, the volume ratio of ethanol to water is any suitable ratio, including but not limited to 1:2 to 1:15.

In other embodiments, the recrystallization herein can be carried out in a mixed solvent of isopropyl acetate and toluene. In some other embodiments, the volume ratio of the isopropyl acetate to toluene can be any suitable ratio, including but not limited to 1:2 to 1:15; specifically, the volume ratio of the isopropyl acetate to toluene can be 1:5.

The purification of the compound of formula (I) described herein can be achieved by multiple recrystallizations, and the conditions of each recrystallization can be the same or different. When necessary, the compound of formula (I) of the present invention can be further removed impurities by other common purification methods in the art, for example, by using palladium-removing silica gel and/or activated carbon to remove residual palladium. The recrystallization can be carried out by dissolving the crude product in a good solvent, stirring and dissolving, then adding a poor solvent, or adding a solution of the crude product to a poor solvent, and stirring to precipitate a solid. The dissolving process can be carried out at normal temperature or under heating. Preferably, after dissolving the crude product in a good solvent, impurities can be removed by means of palladium-removing silica gel and/or activated carbon, and then the obtained solution is added to a poor solvent to precipitate a solid. Preferably, in the above recrystallization, the good solvent can be ethanol or isopropyl acetate, and the poor solvent can be water or toluene. In some embodiments, the good solvent is ethanol, and the poor solvent is water; preferably, the volume ratio of ethanol to water can be from about 1:1.5 to about 1:15, more preferably from about 1:1.8 to about 1:15. In other embodiments, the good solvent is isopropyl acetate, and the poor solvent is toluene; preferably, the volume ratio of isopropyl acetate to toluene can be about 1:2 to about 1:15, more preferably from about 1:4 to about 1:5.

In some embodiments, the compound of formula (II) can be prepared by the method described in the present invention.

In another aspect, the present invention provides a method for preparing a compound of formula (VIII-a), which comprises: reacting a compound of formula (IX) in the presence of lipase to obtain the compound of formula (VIII-a);

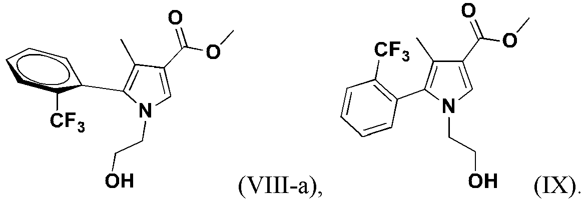

In some embodiments, the lipase is Novozyme 435.

In some embodiments, the solvent of the reaction is *tert*-butyl methyl ether, tetrahydrofuran, toluene, 2-methyltetrahydrofuran, acetone, acetonitrile or ethyl acetate, etc.; preferably, the solvent of the reaction is acetonitrile.

In some embodiments, the reaction is carried out in the presence of an acyl donor such as vinyl propionate.

In some embodiments, the reaction temperature of the reaction is 0°C to 50°C, preferably room temperature or 5°C to 40°C; more preferably, the reaction temperature of the reaction is 5°C to 10°C.

In yet another aspect, the present invention provides a compound of formula (II), formula (III) or formula (VIII-a), 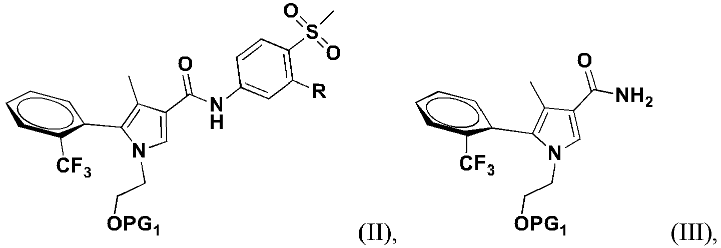 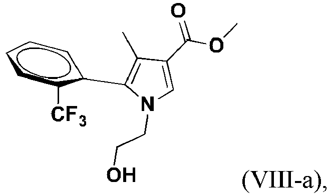 wherein, R has the meaning described in the present invention, and PG₁ is a suitable hydroxyl protecting group, including but not limited to, alkyl, alkenyl, alkoxyalkyl, alkylthioalkyl, silylalkyl, benzyl or substituted benzyl, etc.; preferably, PG₁ is benzyl or substituted benzyl.

In some embodiments, the present invention provides a compound of formula (II-1), formula (II-2) or formula (III-1),

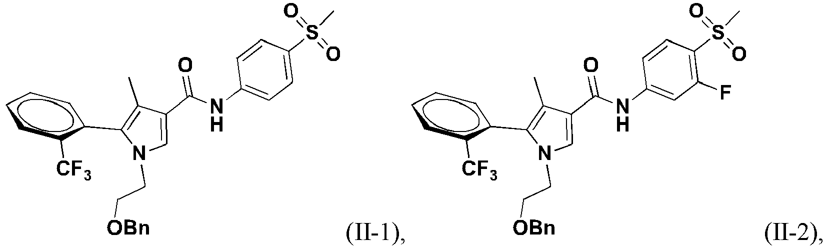

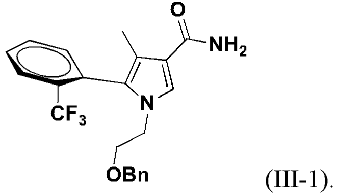

The compound of formula (III-1) of the present invention is used to prepare the compound of formula (IA) or (I) of the present invention. Preferably, the method for preparing the compound of formula (IA) or (I) using the compound of formula (III-1) is as described in the present invention.

The present invention provides a new method for preparing pyrrole amide compounds, wherein the pyrrole amide compounds include Esaxerenone and its analogues. At the same time, the present invention also provides important intermediates in the method and preparation methods thereof. The preparation method of the invention has cheap raw materials, mild conditions, simple operation, safety and controllability, high total yield and high product purity, and is suitable for industrialized production.

### DEFINITIONS AND GENERAL TERMINOLOGY

In the present invention, "room temperature" or "normal temperature" refers to a temperature from about 10°C to about 40°C. In some embodiments, "room temperature" refers to a temperature from about 20°C to about 30°C; in other embodiments, "room temperature" refers to 20°C, 22.5°C, 25°C, 27.5°C, and the like. "r.t." in the present invention is an abbreviation of "room temperature".

"Low temperature" in the present invention refers to a temperature from about 0°C to about 10°C.

In the context of the present invention, all numbers disclosed herein are approximations. The value of each number may vary by 1%, 2%, 5%, 7%, 8% or 10%. When a number with an N value is made public, any number within N +/- 1%, N +/- 2%, N +/- 3%, N +/-5%, N +/- 7%, N +/- 8%, or N +/- 10% will be opened clearly, wherein "+/-" means plus or minus. Whenever a lower limit, DL, and an upper limit, DU, of a numerical range are disclosed, any numerical value within that disclosed range is expressly disclosed.

The "product content" or "product ratio" in the present invention refers to the content of the product in the reaction system detected by HPLC after the reaction is completed.

After the reaction described in the present invention proceeds to a certain extent, such as the raw material is consumed more than 70%, more than 80%, more than 90%, more than 95%, or completely by monitoring, the reaction mixture is post-processed, such as cooled, collected, drawn, filtered, separated, purified or a combination thereof. The reaction can be monitored by conventional methods such as thin-layer chromatography (TLC), high performance liquid chromatography (HPLC), gas chromatography (GC), and the like. The reaction mixture can be post-processed by a conventional method, for example, the crude product can be collected by concentrating the reaction mixture through vacuum evaporation or conventional distillation and is used directly in the next reaction; or the crude product can be obtained by filtration of the reaction mixture and is used directly in the next reaction; or the crude product can be obtained by pouring the supernatant liquid of the reaction mixture after standing and is used directly in the next reaction; or an appropriate organic solvent or a solvent combination is used to carry out purification steps such as extraction, distillation, crystallization, column chromatography, rinsing, and slurrying.

The term "about", "approximately" or "around" in the present invention is used to modify a numerical value with a difference of 10%. In some embodiments, "about", "approximately" or "around" is used to modify a numerical value with a difference of 5%. In some embodiments, "about", "approximately" or "around" is used to modify a numerical value with a difference of 3%, 2% or 1%. It can be understood that the numerical error range modified by "about", "approximately" or "around" depends on the actual or reasonable error range of the modified numerical value.

During each step of the reaction in the present invention, the raw materials or other reagents of the reaction can be added dropwise. Each of the dropping process and each step of the reaction are carried out under certain temperature, and any temperature suitable for each dropping process or each reaction process is included in the present invention. Additionally, many similar modifications in the art, equivalent replacements, or temperature and temperature scope which are equivalent to those described in the invention, all are deemed to be included in the present invention. The present invention provides the preferred temperature or temperature range of each dropwise addition process, and the preferred reaction temperature or reaction temperature range of each reaction.

For the expressions such as "solvent 1", "solvent 2", "solvent a", "solvent b", "base a" and "base b" in the present invention, the Arabic numerals 1, 2, 3... or letters a, b, c... after "solvent" or "base" are only used to better distinguish the solvent or base used in each step, and the Arabic numerals or letters have no special meaning. For example, solvent 1 includes all solvents suitable for the reaction of the compound of formula (II) prepared by reacting the compound of formula (III) with the compound of formula (IV), including but not limited to toluene, dioxane, dimethyl sulfoxide, *tert*-butanol or *tert-*amyl alcohol, dimethyl ether (DME), cyclopentyl methyl ether (CPME), *N*,*N*-dimethylacetamide, water, or any combination thereof.

The solvent used for the reaction of the invention is not particularly limited, and any solvent is contained in the invention so long as it can dissolve the raw materials to a certain extent and doesn't inhibit the reaction. Additionally, many similar modifications in the art, equivalent replacements, or solvents, solvent combinations and solvent combinations with different proportions which are equivalent to those described in the invention, are all deemed to be included in the present invention. The present invention gives the preferred solvent for each reaction step.

The product of each reaction step described in the present invention can be purified by recrystallization under appropriate conditions. The solvent used for the recrystallization is not particularly limited, and any solvent is contained in the invention so long as it can dissolve the crude product and the crystal product can precipitate out under certain conditions. Additionally, many similar modifications in the art, equivalent replacements, or solvents, solvent combinations and solvent combinations with different proportions which are equivalent to those described in the invention, are all deemed to be included in the present invention. Wherein the solvent could be alcohols, ethers, alkanes, halohydrocarbons, esters, ketones, aromatic hydrocarbons, acetonitrile, acetic acid, water, DMF or combinations thereof. Such as water, acetic acid, methanol, ethanol, *n*-propanol, *i*-propanol, *n*-butanol, *i*-butanol, *tert*-butanol, petroleum ether, *n*-pentane, *n*-hexane, *n*-heptane, cyclohexane, DMF, *N*,*N*-dimethylacetamide, tetrahydrofuran, ethyl ether, isopropyl ether, dioxane, methyl tertiary butyl ether (MTBE), 1,2-dimethoxylethane, diethylene glycol dimethyl ether, triethylene glycol dimethyl ether, dichloromethane, 1,2-dichloroethane, chloroform, tetrachloromethane, ethyl acetate, isopropyl acetate, acetone, butanone, benzene, toluene, xylene or combinations thereof.

The amount of water in the solvent is not particularly limited, that is, the amount of water in the solvent does not affect the occurrence of the reaction in the present invention. So long as the solvent containing a certain amount of water can be used in the reaction disclosed herein, which is deemed to be included in the present invention. For example, the amount of water in the solvent is approximately less than 0.05%, less than 0.1%, less than 0.2%, less than 0.5%, less than 5%, less than 10%, less than 25%, less than 30%, or 0%. In some embodiments, the amount of water in the solvent is within a certain range, which is more conducive to the reaction; for example, in the step of using ethanol as the reaction solvent, using absolute ethanol is more conducive to the reaction. In some embodiments, the amount of water in the solvent exceeds a certain range, which may affect the progress of the reaction (for example, affect the yield of the reaction), but does not affect the occurrence of the reaction.

"Substituted" in the present invention means that the group can be substituted by at least one reasonable substituent, including but not limited to: halogen, deuterium, C₁₋₂₀ alkyl, C₁₋₂₀ alkoxy, C₁₋₂₀ haloalkyl or C₁₋₂₀ haloalkoxy, preferably halogen, deuterium, C₁₋₄ alkyl, C₁₋₄ alkoxy, C₁₋₄ haloalkyl or C₁₋₄ haloalkoxy. For example, "substituted benzyl" may be *p*-methylbenzyl, *p*-methoxybenzyl, and the like.

Abbreviations and meanings appearing in the present invention are as follows:

| Abbreviations | Meanings |
|---|---|
| Bn | Benzyl |
| CDI | *N*,*N*'-Carbonyldiimidazole |
| Pd₂(dba)₃ | Tris(dibenzylideneacetone)dipalladium |
| Pd(dppf)Cl₂·CH₂Cl₂ | 1,1'-bis(diphenylphosphino)ferrocene-palladium(II)dichloride dichloromethane complex |
| Xantphos | 4,5-bis(diphenylphosphine)-9,9-dimethylxanthene |
| S-phos | 2-Dicyclohexylphosphino-2',6'-dimethoxybiphenyl |
| X-phos | 2-(Dicyclohexylphosphino)-2',4',6'-triisopropylbiphenyl |
| DMEDA | *N*,*N*'-Dimethylethylenediamine |
| TMEDA | *N*,*N*,*N*',*N*'-Tetramethylethylenediamine |
| CPME | Cyclopentyl methyl ether |
| DME | Dimethyl ether |
| t-AmOH | *Tert*-amyl alcohol |
| t-BuOH | *Tert*-butanol |
| DMAC | *N*,*N*-dimethylacetamide |
| DMF | *N,N-*dimethylformamide |
| THF | Tetrahydrofuran |
| DMSO | Dimethyl sulfoxide |
| NMP | *N*-methylprrolidone |

### General Synthesis and Determination Methods

In the present invention, if the chemical name of the compound doesn't match the corresponding structure, the compound is characterized by the corresponding structure.

In the examples described below, all temperatures are set forth in degrees Celsius (°C) unless otherwise indicated. Unless otherwise indicated, various materials and reagents were purchased from commercial suppliers and without further purification; some materials can be prepared according to the methods known in the art.

¹H NMR spectra were recorded by a Bruker Avance 400 MHz spectrometer or Bruker Avance III HD 600 spectrometer, using CDCl₃, *d*₆-DMSO, CDeOD, D₂O or *d*₆-acetone (reported in ppm) as solvent, and using TMS (0 ppm) or chloroform (7.25 ppm) as the reference standard. When peak multiplicities are reported, the following abbreviations are used: s (singlet), d (doublet), t (triplet), m (multiplet), br (broadened), dd (doublet of doublets), dt (doublet of triplets), td (triplet of doublets), ddd (doublet of doublet of doublets), ddt (doublet of doublet of triplets), dddd (doublet of doublet of doublet of doublets). Coupling constants, when given, were reported in Hertz (Hz).

The purity and/or enantiomeric excess of each compound can be determined according to the chromatographic analysis method described in the present invention. The chromatographic conditions in the present invention are not limited, and can be properly selected or adjusted according to the separation effect of the compounds and the impurities or enantiomers.

The embodiments of the present invention disclose a method for preparing the pyrrole amide compound of formula (IA), formula (I), formula (IIA) or formula (II). Skilled in the art can learn from this article to properly improve the process parameters to implement the preparation method. Of particular note is that all similar substitutions and modifications to the skilled person is obvious, and they are deemed to be included in the present invention. The method of the present invention has been described through preferred embodiments, and related person can clearly realize and apply the techniques disclosed herein by making some changes, appropriate alterations or combinations to the methods without departing from spirit, principles and scope of the present disclosure.

In order to enable those skilled in the art to further understand the invention, it is detailed below through examples.

### Example

### Example 1 Synthesis of intermediate (S)-1-(2-(benzyloxy)ethyl)-4-methyl-5-(2-(trifluoromethyl)phenyl)-1H-pyrrole-3-carboxamide

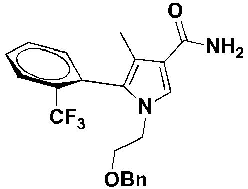

### Step 1: Methyl (S)-1-(2-hydroxyethyl)-4-methyl-5-(2-(trifluoromethyl)phenyl)-1H-pyrrole-3-carboxylate

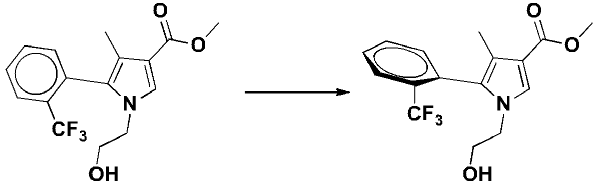

Vinyl propionate (1880 mL, 16900 mmol) and Novozyme 435 lipase (92.2 g) were added to a solution of methyl 1-(2-hydroxyethyl)-4-methyl-5-(2-(trifluoromethyl)phenyl)-1*H*-pyrrole-3-carboxylate (3073 g, 9389 mmol) in acetonitrile (30.7 L), then the mixture was stirred at room temperature and the reaction was monitored by HPLC. The reaction was stopped after 3.5 h. The reaction solution was filtered to remove lipase, the filtrate was concentrated under vacuum, and the residue was separated by silica gel column chromatography (petroleum ether/ethyl acetate (v/v)=1/1) to obtain light yellow transparent oil (841 g, 27.37%). HPLC purity was 97.03%, and *ee* value was 93.84%.

The purity of the target compound was determined by high performance liquid chromatography:
Blank solution: methanol; test solution: the test sample was taken and prepared to a solution with a concentration of about 1 mg/mL by using methanol. Mobile phase A: 0.05% phosphoric acid aqueous solution; mobile phase B: acetonitrile; elution procedure: gradient elution (mobile phase A: mobile phase B = 9: 1~2:8). Chromatographic column: Xbridge Phenyl (4.6×150 mm, 3.5 µm); flow rate: 1.0 mL/min; detection wavelength: 238 nm; column temperature: 45°C; injection volume: 4 µL; and stop time: 40 min.

The enantiomeric excess of the target compound was determined by the following method:
Blank solution: ethanol; test solution: the test sample was taken and prepared to a solution with a concentration of about 1 mg/mL by using ethanol. Mobile phase A: 1% trifluoroacetic acid-ethanol solution; mobile phase B: *n*-hexane; elution procedure: isocratic elution (mobile phase A: mobile phase B = 3:97). Chromatographic column: CHIRAL AS-H (4.6 mm×250 mm, 5 µm); flow rate: 0.7 mL/min; detection wavelength: 238 nm; column temperature: 25°C; injection volume: 3 µL; and stop time: 50 min.

### Step 2: Methyl (S)-1-(2-(benzyloxy)ethyl)-4-methyl-5-(2-(trifluoromethyl)phenyl)-1H-pyrrole-3-carboxylate

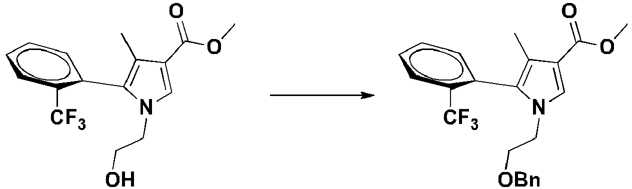

Methyl (*S*)-1-(2-hydroxyethyl)-4-methyl-5-(2-(trifluoromethyl)phenyl)-1*H*-pyrrole-3-carboxylate (1580 g, 4827 mmol) and benzyl bromide (1432 g, 8205 mmol) were added to DMAC (31600 mL), the mixture was cooled to 10 °C, and then sodium *tert*-pentoxide (922.2 g, 8206 mmol) was added in batches. The air in the reactor was replaced with N₂ and the temperature was raised to 40°C, and the reaction was stopped after stirring for 4h. The reaction solution was poured into ice water (50 L) and extracted with ethyl acetate (5 L × 2). The organic phases were combined and washed with saturated brine (10 L × 2), then the organic phase was collected and concentrated under vacuum to obtain brown oil (1960 g, 97.27%).

The purity of the target compound or the content of the product is determined by using high performance liquid chromatography:
Blank solution: methanol; test solution: the test sample was taken and diluted 10 times with methanol. Mobile phase A: 0.05% phosphoric acid aqueous solution; mobile phase B: acetonitrile; elution procedure: gradient elution (mobile phase A: mobile phase B = 8:2~2:8). Chromatographic column: Xbridge Phenyl (4.6×150 mm, 3.5 µm); flow rate: 1.0 mL/min; detection wavelength: 238 nm; column temperature: 40°C; injection volume: 1 µL; and stop time: 40 min.

Referring to the above method, methyl (*S*)-1-(2-hydroxyethyl)-4-methyl-5-(2-(trifluoromethyl)phenyl)-1*H*-pyrrole-3-carboxylate was used as a raw material, the conditions such as the type and amount of solvent, the type and amount of base, the amount of benzyl bromide, and the reaction temperature were screened. Results are as shown in table A. It can be seen from Table A that different bases or different solvents have a great impact on the reaction.

**Table A**

| **Embodiments** | **Benzyl bromide** | **Base** | **Solvent** | **Reaction temperature** | **Product content** |
|---|---|---|---|---|---|
| 1 | 2.0 eq. | Potassium ethoxide (2.0 eq.) | DMF | 90 °C | 0 |
| 2 | 4.0 eq. | Sodium methoxide (4.0 eq.) | DMF | 60-90 °C | 57.22% |
| 3 | 2.0 eq. | Sodium ethoxide (2.0 eq.) | DMF | 60 °C | 87.59% |
| 4 | 2.5 eq. | Potassium hydroxide (2.5 eq.) | DMF | 60-90 °C | 84.54% |
| 5 | 1.3 eq. | Sodium hydroxide (1.7 eq.) | DMF (10 mL/g) | r.t.-80°C | 76.67% |
| 6 | 2.0 eq. | Sodium *tert*-pentoxide (2.0 eq.) | DMF | 60 °C | 95.18% |
| 7 | 4.0 eq. | Tetrabutylammonium hydroxide (4.0 eq.) | DMF | 60-90 °C | 73.59% |
| 8 | 1.7 eq. | DBU (1.7 eq.) | DMF (10 mL/g) | 80 °C | 1.22% |
| 9 | 2.0 eq. | Sodium acetate (2.0 eq.) | DMF | 90 °C | 0 |
| 10 | 2.0 eq. | Potassium phosphate (2.0 eq.) | DMF | 60 °C | 0 |
| 11 | 1.5 eq. | Cesium carbonate (1.5 eq.) | DMF | 90 °C | 2.45% |
| 12 | 1.0 eq. | Sodium *tert*-pentoxide | DMF (10 mL/g) | 60 °C | 88.28% |
| | | (1.0 eq.) | | | |
| 13 | 1.1 eq. | Sodium *tert*-pentoxide (1.1 eq.) | DMF (10 mL/g) | 60 °C | 95.60% |
| 14 | 1.2 eq. | Sodium *tert*-pentoxide (1.2 eq.) | DMF (10 mL/g) | 60 °C | 95.11% |
| 15 | 1.5 eq. | Sodium *tert*-pentoxide (1.5 eq.) | DMF (10 mL/g) | 60 °C | 95.55% |
| 16 | 2.5 eq. | Sodium *tert*-pentoxide (2.5 eq.) | DMF | 60 °C | 90.29% |
| 17 | 1.1 eq. | Sodium *tert*-pentoxide (1.1 eq.) | DMF | 40 °C | 97.23% |
| 18 | 1.1 eq. | Sodium *tert*-pentoxide (1.1 eq.) | DMF | r.t. | 95.45% |
| 19 | 1.7 eq. | Sodium *tert*-pentoxide (1.7 eq.) | Toluene (10 mL/g) | 50 °C | 26.76% |
| 20 | 1.7 eq. | Sodium *tert*-pentoxide (1.7 eq.) | THF (10 mL/g) | 50 °C | 50.77% |
| 21 | 1.7 eq. | Sodium tert-pentoxide (1.7 eq.) | DMSO (10 mL/g) | 50 °C | 63.45% |
| 22 | 1.7 eq. | Sodium *tert*-pentoxide (1.7 eq.) | NMP (10 mL/g) | 50 °C | 51.33% |
| 23 | 1.7 eq. | Sodium *tert*-pentoxide (1.7 eq.) | DMAC | 50 °C | 96.99% |
| 24 | 1.2 eq. | Sodium *tert*-pentoxide (1.2 eq.) | DMAC | 60 °C | 98.12% |
| 25 | 1.05 eq. | Sodium *tert*-pentoxide (1.05 eq.) | DMAC | 40 °C | 92.17% |
| 26 | 1.05 eq. | Sodium *tert*-pentoxide (1.1 eq.) | DMAC | 40 °C | 94.58% |
| 27 | 1.05 eq. | Sodium *tert*-pentoxide (1.1 eq.) | DMAC | 40 °C | 84.35% |
| 28 | 1.1 eq. | Sodium *tert*-pentoxide (1.1 eq.) | DMAC | 40 °C | 96.79% |
| 29 | 1.1 eq. | Sodium *tert*-pentoxide (1.2 eq.) | DMAC | 40 °C | 86.79% |
| 30 | 1.1 eq. | Sodium *tert*-pentoxide (1.05 eq.) | DMAC | 40 °C | 90.85% |
| 31 | 1.1 eq. | Sodium *tert*-pentoxide (1.1 eq.) | DMAC (10 mL/g) | 90 °C | 95.49% |
| 32 | 1.1 eq. | Sodium *tert*-pentoxide (1.1 eq.) NaI (0.1 eq.) | DMAC | 40 °C | 98.50% |
| 33 | 1.3 eq. | Sodium *tert*-pentoxide (1.3 eq.) | DMAC | 30°C | 97.77% |
| 34 | 1.3 eq. | Sodium *tert*-pentoxide (1.3 eq.) | Acetonitrile | 50 °C | 88.31% |
| 35 | 1.3 eq. | Sodium *tert*-pentoxide (1.3 eq.) | DMAC | 50 °C | 98.34% |

| | | | | | |
|---|---|---|---|---|---|
| Note: Equivalent or eq. refers to the ratios of molar amounts of other materials based on the molar amount of methyl (*S*)-1-(2-hydroxyethyl)-4-methyl-5-(2-(trifluoromethyl)phenyl)-1*H*-pyrrole-3-carboxylate. | | | | | |

Embodiment 27 was carried out without N₂.

Unless otherwise stated, the amount of solvent in Table A was generally 20 mL/g, that is, every 1 g of methyl (*S*)-1-(2-hydroxyethyl)-4-methyl-5-(2-(trifluoromethyl)phenyl)-1*H*-pyrrole-3-carboxylate, 20 mL of solvent was used.

### Step 3: (S)-1-(2-(benzyloxy)ethyl)-4-methyl-5-(2-(trifluoromethyl)phenyl)-1H-pyrrole-3-carboxylic acid

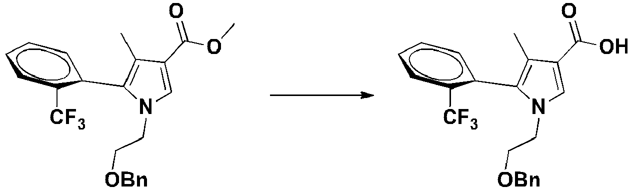

Potassium hydroxide (1240 g, 18786 mmol, 85 mass%) was slowly added to a solution of methyl (S)-1-(2-(benzyloxy)ethyl)-4-methyl-5-(2-(trifluoromethyl)phenyl)-1*H*-pyrrole-3-carboxylate (1960 g, 4696 mmol) in a mixed solvent of methanol (7840 mL) and water (1960 mL), the mixture was heated to 70 °C, condensed to reflux, and stirred for 4 h, then the reaction was stopped. The solvent was removed by concentration under vacuum, then the resulting solution was diluted with water (8 L) and washed with a mixed solvent of methyl *tert*-butyl ether (8 L) and petroleum ether (4 L), the obtained aqueous phase was poured into a solution of concentrated hydrochloric acid (2000 mL, 12 mol/L) in water (15 L) at 10 °C, the mixture was slurried overnight and filtered with suction, and the filter cake was dried to obtain a pale yellow solid (1785 g, 94.23%). HPLC purity was 99.38%.

The purity of the target compound was determined by high performance liquid chromatography:
Blank solution: methanol; test solution: the test sample was taken and prepared to a solution with a concentration of about 0.8 mg/mL by using ethanol. Mobile phase A: 0.05% phosphoric acid aqueous solution; mobile phase B: acetonitrile; elution procedure: gradient elution (mobile phase A: mobile phase B = 8:2-1.5:8.5). Chromatographic column: Xbridge Phenyl (4.6×150 mm, 3.5 µm); flow rate: 1.0 mL/min; detection wavelength: 220 nm; column temperature: 30°C; injection volume: 7 µL; and stop time: 36 min.

### Step 4: (S)-1-(2-(benzyloxy)ethyl)-4-methyl-5-(2-(trifluoromethyl)phenyl)-1H-pyrrole-3-carboxamide

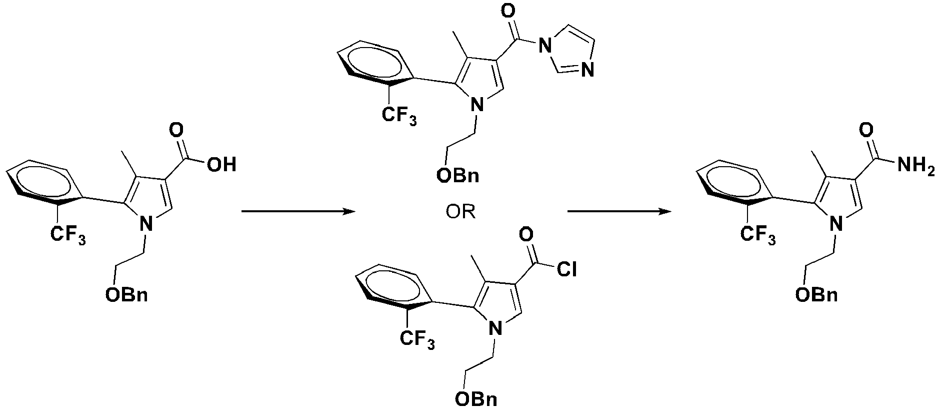

### Method one:

### Embodiment 1:

N,N-Dimethylformamide (260 mL) was added to (*S*)-1-(2-(benzyloxy)ethyl)-4-methyl-5-(2-(trifluoromethyl)phenyl)-1*H*-pyrrole-3-carboxylic acid (129.5 g , 321.0 mmol) in a 1000 mL flask, then *N*,*N*'-carbonyldiimidazole (74.6 g, 451 mmol, 98 mass%) was added in batches, the mixture was reacted at room temperature for 2 h. Under ice-bath condition, the above reaction solution was added to ammonium hydroxide (520 mL, 3460 mmol, 26 mass%), and then the mixture was reacted at room temperature for 15 h. Then dilute hydrochloric acid (1 M, 520 mL) was added, and the mixture was continuously reacted at room temperature for 3 h. After suction filtration, the filter cake was washed with water (100 mL × 2), collected and dried under vacuum at 60°C for 12 h to obtain a crude product as an off-white solid (127 g, 98.31%).

The crude product (10 g) was weighed into a 100 mL flask, to which was added tert-butyl acetate (30 mL), and the mixture was heated to 85 °C, then cyclohexane (30 mL) was added to the mixture, and the heating was turned off after the resulting mixture was stirred at 85 °C for 2 h. The resulting mixture was continuously stirred for 3 h after cooling to room temperature. After suction filtration, the filter cake was washed with cyclohexane/*tert*-butyl acetate ((v/v) = 1/1, 20 mL), collected, and dried under vacuum at 60°C to obtain an off-white solid (9.37 g, 93.7%). HPLC purity was 99.84%, and *ee* value was 100%.

The purity of the target compound was determined by high performance liquid chromatography:
Blank solution: methanol; test solution: the test sample was taken and prepared to a solution with a concentration of about 1.0 mg/mL by using methanol. Mobile phase A: 0.05% ammonium hydroxide-water solution (phosphoric acid was used to adjust pH to 8); mobile phase B: acetonitrile; elution procedure: gradient elution (mobile phase A: mobile phase B = 7:3~2:8). Chromatographic column: ZORBAX Extend-C18 (4.6×150mm, 5µm); flow rate: 1.0 mL/min; detection wavelength: 238 nm; column temperature: 30°C; injection volume: 6 µL; and stop time: 25 min.

The enantiomeric excess of the target compound was determined by the following method:
Blank solution: methanol; test solution: the test sample was taken and prepared to a solution with a concentration of about 1 mg/mL by using ethanol. Mobile phase A: 1% trifluoroacetic acid-ethanol solution; mobile phase B: *n*-hexane; elution procedure: isocratic elution (mobile phase A: mobile phase B = 1:9). Chromatographic column: CHIRALPAK AS-H (4.6×250mm, 5µm); flow rate: 1.0 mL/min; detection wavelength: 238 nm; column temperature: 30°C; injection volume: 6 µL; and stop time: 40 min.

### Embodiment 2:

CDI (952 g, 5754 mmol) was added in batches to a solution of (*S*)-1-(2-(benzyloxy)ethyl)-4-methyl-5-(2-(trifluoromethyl)phenyl)-1*H*-pyrrole-3-carboxylic acid (1785 g, 4425 mmol) in DMF (3570 mL) at room temperature, the mixture was stirred for 1 h at room temperature and then the reaction was stopped. The reaction solution was directly used in the next step, and the yield was calculated as 100%.

At 10°C, the reaction solution of (*S*)-(1-(2-(benzyloxy)ethyl)-4-methyl-5-(2-(trifluoromethyl)phenyl)-1*H*-pyrrol-3-yl)(1*H*-imidazol-1 -yl)methanone (2007 g, 4426 mmol) in DMF (3570 mL) prepared as above was slowly added to ammonium hydroxide (7000 mL, 26 mass%), and the mixture was stirred at 10 °C for 3 h, then slowly warmed to room temperature and stirred overnight. To the mixture was added hydrochloric acid (8 L, 1.5 mol/L), and the resulting mixture was stirred for 1 h. After suction filtration, the filter cake was dried to obtain a crude product as an off-white solid (1700 g, 95.46%).

The obtained crude product (1700 g, 4225 mmol) was dissolved in isopropyl acetate (5100 mL), the mixture was heated to about 80 °C and stirred for 30 min until the system became clear, to which was added cyclohexane (5100 mL) and the resulting mixture was stirred for 1 h. The heating was turned off and the mixture was stirred overnight. After suction filtration, an off-white solid (1510g, 88.8%) was obtained, the *ee* value was 99.44% and the HPLC purity was 99.97%.

When the volume ratio of cyclohexane to isopropyl acetate was 2:1, or, isopropyl acetate was replaced with *tert*-butyl acetate, the purification effect can also be achieved. For example, isopropyl acetate was replaced with *tert*-butyl acetate for recrystallization and purification (cyclohexane/*tert*-butyl acetate (v/v) = 3/4, the amount was 7 mL of mixed solvent per gram of the crude product), the HPLC purity of the obtained product was 99.94%, and the *ee* value was 99.78%.

### Method two:

*N*,*N*-Dimethylformamide (20 mL) and *N*,*N*'-carbonyldiimidazole (4.92 g, 29.76 mmol) were added to (*S*)-1-(2-(benzyloxy)ethyl)-4-methyl-5-(2-(trifluoromethyl)phenyl)-1*H*-pyrrole-3-carboxylic acid (10.0 g , 24.8 mmol) in a 100 mL flask, and then the mixture was reacted at room temperature for 2 h. To the flask were added ammonium thiocyanate (7.65 g, 99.0 mmol) and potassium carbonate (12.8 g, 91.7 mmol), and the mixture was reacted at room temperature for 12 h. Water (80 mL) was added to the flask, and the mixture was stirred at room temperature for 2 h. After suction filtration, the filter cake was washed with water (20 mL × 2), collected, and dried under vacuum at 60°C for 12 h to obtain an off-white solid (9.50 g, 95.2%), the HPLC purity was 99.21% and the *ee* value was 99.02%.

### Method three:

*N*,*N*'-Carbonyldiimidazole (4.92 g, 29.76 mmol) and *N*,*N*-dimethylformamide (20 mL) were added to (*S*)-1-(2-(benzyloxy)ethyl)-4-methyl-5-(2-(trifluoromethyl)phenyl)-1*H*-pyrrole-3-carboxylic acid (10.0 g , 24.8 mmol) in a 100 mL flask, and the mixture was reacted at room temperature for 2 h . To the flask were added ammonium bromide (12.2 g, 123 mmol) and potassium carbonate (12.8 g, 91.7 mmol), and the mixture was reacted at room temperature for 17 h. Water (80 mL) was added to the flask and the mixture was stirred at room temperature for 30 min. After suction filtration, the filter cake was washed with water (10 mL × 3), collected, and dried under vacuum at 60°C for 12 h to obtain an off-white solid (9.40 g, 94.6%), the HPLC purity was 98.92%, and the *ee* value was 98.64%; MS (ESI, pos. ion) m/z: 403.2 (M+1).

Referring to the above method two and method three, the product is prepared with different amination reagents or different amounts of amination reagents, and the results are shown in Table B. It can be seen from Table B that different amination reagents have a great influence on the reaction. For example, under the same conditions, the yield is low when ammonium chloride or ammonium bicarbonate is used as the amination reagent to prepare the target amide compound.

**Table B**

| **Embodiments** | **Amination reagent** | **Amount** | **Yield** |
|---|---|---|---|
| 1 | NH₄Cl | 5 eq. | 40% |
| 2 | NH₄Br | 3 eq. | 93.5% |
| 3 | NH₄HCO₃ | 5 eq. | 40% |
| 4 | NH₃·H₂O | 10 eq. | 98.3% |

| | | | |
|---|---|---|---|
| Note: Equivalent or eq. refers to the ratios of molar amounts of other materials based on the molar amount of (*S*)-1-(2-(benzyloxy)ethyl)-4-methyl-5-(2-(trifluoromethyl)phenyl)-1*H*-pyrrole-3-carboxylic acid. | | | |

### Method four:

Dichloromethane (100 mL) and 3 drops of *N*,*N*-dimethylformamide were added to (*S*)-1-(2-(benzyloxy)ethyl)-4-methyl-5-(2-(trifluoromethyl)phenyl)-1*H*-pyrrole-3-carboxylic acid (10 g, 24.79 mmol) in a 250 mL flask, to the mixture was dropwise added oxalyl chloride (3.2 mL, 38 mmol) under ice-bath, then the mixture was reacted at room temperature for 2 h. The solvent was removed by concentration under vacuum, and then the mixture was dissolved in tetrahydrofuran (50 mL) completely. The above solution was added dropwise to ammonium hydroxide (100 mL, 1330 mmol, 25 mass%) under ice-bath, and then the resulting mixture was reacted at room temperature for 10 h. Water (100 mL) was added to the system, and the mixture was stirred at room temperature for 3 h. After suction filtration, the filter cake was washed with water (20 mL), collected and dried under vacuum at 60°C for 12 h to obtain a pale yellow crude product.

To the crude product was added isopropyl acetate (28.5 mL), the mixture was heated to 80°C and then to which was added dropwise cyclohexane (57 mL), the mixture was continuously stirred at 80°C for 1 h, and then the heating was turned off. After cooling to room temperature, the mixture was continuously stirred for 15 h. After suction filtration, the filter cake was washed with cyclohexane/isopropyl acetate ((v/v) = 2/1, 28 mL), then collected and dried under vacuum at 60°C for 12 h to obtain an off-white solid (9.39 g, 94 %), the HPLC purity was 99.75%, and the *ee* value was 100%; MS (ESI, pos. ion) m/z: 403.2 (M+1).

### Example 2 Synthesis of (S)-N-(3-fluoro-4-(methylsulfonyl)phenyl)-1-(2-hydroxyethyl)-4-methyl-5-(2-(trifluoromethyl) phenyl)-1H-pyrrole-3-carboxamide

### Step 1: (S)-1-(2-(benzyloxy)ethyl)-N-(3-fluoro-4-(methylsulfonyl)phenyl)-4-methyl-5-(2-(trifluorometh yl)phenyl)-1H-pyrrole-3-carboxamide

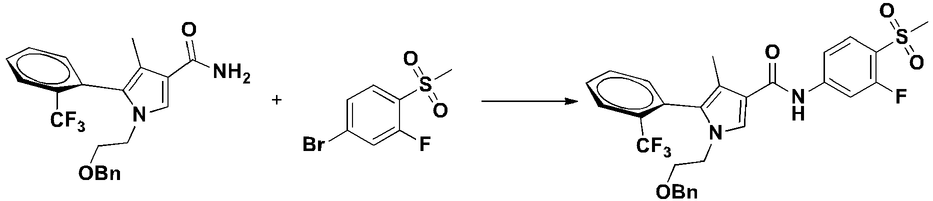

To (*S*)-1-(2-(benzyloxy)ethyl)-4-methyl-5-(2-(trifluoromethyl)phenyl)-1*H*-pyrrole-3-carboxamide (2.12 kg, 5.268 mol) were added *tert*-amyl alcohol (13.8 kg), 4-bromo-2-fluoro-1-(methylsulfonyl)benzene (1.38 kg, 5.453 mol), cesium carbonate (3.45 kg, 10.59 mol), palladium acetate (31.8 g, 141.6 mmol) and 4,5-bisdiphenylphosphine-9,9-dimethylxanthene (Xantphos) (77.0 g, 133.0 mmol), the reaction system was replaced with N₂ 3 times, and then heated to 65°C and reacted for 4 h. After the reaction was stopped, to the system was added water (10.90 kg) and the mixture was stirred for 5 min, then isopropyl acetate (5.93 kg) was added to the reaction solution for dilution, and the mixture was stirred for 5 min, then stood and separated. The lower aqueous phase was separated, the upper organic phase was filtered through a celite pad, and the filter cake was washed with isopropyl acetate (5.89 kg). The filtrate was concentrated under vacuum, then ethanol (9.95 kg) was added to the concentrate. At 10°C, the above mixed solution was dropwise added to water (91.20 kg), and the mixture was continuously stirred for 5 h. After suction filtration, the filter cake was washed with water (4.85 kg), then collected and dried under vacuum at 45°C for 48 h to obtain a crude product.

Ethanol (9.30 kg) was added to the crude product to dissolve it, then to which were added palladium-removing silica gel (470 g) and activated carbon (170 g), the mixture was heated to 70 °C and stirred for 1 h, then the heating was turned off and the mixture was cooled to room temperature and stirred for 12 h. The resulting mixture was filtered through a celite pad, the filter cake was washed with ethanol (4.65 kg), and the filtrate was collected. At 10°C, the above filtrate was added dropwise to water (91.55 kg), and then the mixture was stirred for 5 h. After suction filtration, the filter cake was washed with water (5.50 kg), then collected and dried under vacuum at 45°C for 48 h to obtain an off-white solid (2.95 kg, 97.4%), the HPLC purity was 95.0%, the *ee* value was 98.8%, and the content of Pd was 55.68 ppm.

The purity of the target compound was determined by high performance liquid chromatography:
Blank solution: methanol; test solution: the test sample was taken and prepared to a solution with a concentration of about 1.0 mg/mL by using ethanol. Mobile phase A: 1.36 g/L potassium dihydrogen phosphate aqueous solution (phosphoric acid was used to adjust the pH to 2.0); mobile phase B: acetonitrile; elution procedure: gradient elution (mobile phase A: mobile phase B = 6.5:3.5~2:8). Chromatographic column: Xbridge Phenyl (4.6×150 mm, 3.5 µm); flow rate: 1.0 mL/min; detection wavelength: 210 nm; column temperature: 35°C; injection volume: 4 µL; and stop time: 35 min.

The enantiomeric excess of the target compound was determined by the following method:
Blank solution: ethanol; test solution: the test sample was taken and prepared to a solution with a concentration of about 2 mg/mL by using ethanol. Mobile phase A: 1% trifluoroacetic acid-ethanol solution; mobile phase B: *n*-hexane; elution procedure: isocratic elution (mobile phase A: mobile phase B = 3:7). Chromatographic column: CHIRAL NY (4.6×250 mm, 5 µm); flow rate: 1.0 mL/min; detection wavelength: 290 nm; column temperature: 30°C; injection volume: 6 µL; and stop time: 40 min.
(1) Referring to the above method, the reaction conditions such as the catalyst, ligand, base, solvent and reaction temperature were screened. The experimental results are shown in Table C below. As can be seen from Table C, when using CuI as a catalyst and using commonly ligand such as 8-hydroxyquinaldine, DMEDA or TMEDA, the reaction does not proceed substantially or the reaction effect is extremely poor; while using the palladium catalyst and appropriate ligand of the present invention, the reaction effect is better. In addition, the base has an effect on the reaction, for example, when using CF₃COONa, the reaction effect is poor.

**Table C**

| **Embodi ments** | **Catalyst** | **Ligand** | **Base** | **Solvent** | **Temperatu re** | **Product Content** |
|---|---|---|---|---|---|---|
| 1 | CuI (20%) | 8-Hydroxyquinaldi ne (20%) | K₂CO₃ | Toluene | 110°C | N/A |
| 2 | Pd(OAc)₂(3%) | Xantphos(3%) | CF₃COON a | *t*-BuOH | 80 °C | 0.20% |
| 3 | CuI (20%) | DMEDA (20%) | K₃PO₄ | Toluene | 110°C | 2.71% |
| 4 | CuI (20%) | TMEDA (20%) | K₃PO₄ | Toluene | 110°C | N/A |
| 5 | Pd(OAc)₂(3%) | Xantphos(3%) | K₃PO₄ | *t*-BuOH | 80 °C | 95.4% |
| 6 | Pd(OAc)₂(3%) | Xantphos(3%) | K₂CO₃ | *t*-BuOH | 80 °C | 98.6% |
| 7 | Pd₂(dba)₃(3%) | Xantphos(3%) | Cs₂CO₃ | Dioxan e | 100 °C | 96.5% |
| 8 | Pd₂(dba)₃(3%) | X-phos(3%) | Cs₂CO₃ | Dioxan e | 100 °C | 96.4% |
| 9 | Pd₂(dba)₃(3%) | S-phos(3%) | Cs₂CO₃ | Dioxan e | 100 °C | 92.9% |
| 10 | Pd(dppf)Cl₂·CH₂ Cl₂(3%) | Xantphos(3%) | Cs₂CO₃ | Dioxan e | 100 °C | 97.2% |
| 11 | Pd(dppf)Cl₂·CH₂ Cl₂(3%) | None | Cs₂CO₃ | *t*-BuOH | 100 °C | 33.0% |
| 12 | Pd(OAc)₂(3%) | Xantphos(3%) | Cs₂CO₃ | Dioxan e | 100 °C | 97.5% |
| 13 | Pd(OAc)₂(3%) | Xantphos(3%) | Cs₂CO₃ | *t*-AmO H | 80 °C | 98.4% |
| 14 | Pd(OAc)₂(3%) | Xantphos(3%) | Cs₂CO₃ | DME | 80 °C | 97.9% |
| 15 | Pd(OAc)₂(3%) | X-phos(3%) | Cs₂CO₃ | *t*-BuOH | 80 °C | 98.9% |
| 16 | Pd(OAc)₂(3%) | S-phos(3%) | Cs₂CO₃ | *t*-AmO H | 80 °C | 86.0% |
| 17 | Pd(OAc)₂(3%) | Xantphos(3%) | Cs₂CO₃ | Toluene | 80 °C | 97.1% |
| 18 | Pd(OAc)₂(3%) | Xantphos(3%) | Cs₂CO₃(1.5eq.) | *t*-BuOH | 80 °C | 98.5% |
| 19 | Pd(OAc)₂(3%) | Xantphos(3%) | Cs₂CO₃ | *t*-BuOH | 78 °C | 99.4% |
| 20 | Pd(OAc)₂(3%) | Xantphos(3%) | Cs₂CO₃ | *t*-BuOH /H₂O | 78 °C | 80% |
| 21 | Pd(OAc)₂(3%) | Xantphos(3%) | Cs₂CO₃ | *t*-BuOH | 67 °C | 92.8% |
| 22 | Pd(OAc)₂(3%) | Xantphos(3%) | Cs₂CO₃ | CPME | 78 °C | 97.8% |
| 23 | Pd(OAc)₂(2%) | Xantphos(2%) | Cs₂CO₃ | *t*-BuOH | 78 °C | 97.30% |
| 24 | Pd(OAc)₂(2%) Bromine substrate (1.03 eq) | Xantphos(2%) | Cs₂CO₃ | *t*-BuOH | 78 °C | 98.27% |
| 25 | Pd(OAc)₂(1.5%) | Xantphos(1.5%) | Cs₂CO₃ | *t*-BuOH | 78 °C | 91.02% |
| 26 | Pd(OA_{C})₂(1%) | Xantphos(3%) | Cs₂CO₃ | *t*-BuOH | 78 °C | 67.36% |
| 27 | Pd(OAc)₂(2.5%) | Xantphos(2.5%) | Cs₂CO₃ | *t*-BuOH | 78 °C | 98.54% |
| 28 | Pd(OAc)₂(2.5%) Bromine substrate (1.05 eq) | Xantphos(2.5%) | Cs₂CO₃ | *t*-BuOH | 78 °C | 98.14% |
| 29 | Pd(OAc)₂(2.5%) Bromine substrate (1.01 eq) | Xantphos(2.5%) | Cs₂CO₃ | *t*-BuOH | 78 °C | 96.75% |
| 30 | Pd(OAc)₂(2.5%) | Xantphos(2.5%) | Cs₂CO₃ | *t*-BuOH | 83 °C | 96.78% |
| 31 | Pd(OAc)₂(2.5%) | Xantphos(2.5%) | Cs₂CO₃ | t-BuOH | 73 °C | 97.81% |
| 32 | Pd(OAc)₂(2.5%) | Xantphos(2.5%) | Cs₂CO₃ | t-BuOH | 68 °C | 97.93% |
| 33 | Pd(OAc)₂(2.5%) | Xantphos(2.5%) | Cs₂CO₃ | t-BuOH | 63 °C | 97.21% |
| 34 | Pd(OAc)₂(2.5%) | Xantphos(2.5%) | Cs₂CO₃ | t-BuOH | 58 °C | 97.86% |
| 35 | Pd(OAc)₂(2.5%) | Xantphos(2.5%) | Cs₂CO₃ | t-BuOH | 48 °C | 97.27% |
| 36 | Pd(OAc)₂(2.5%) | Xantphos(2.5%) | Cs₂CO₃ | *t*-AmO H (8 mL/g) | 65 °C | 98.24% |
| 37 | Pd(OAc)₂(2.5%) | Xantphos(2.5%) | Cs₂CO₃ | *t*-AmO H (8 mL/g) | 60 °C | 98.55% |

| | | | | | | |
|---|---|---|---|---|---|---|
| Note: N/A means no product or no product detected. eq., refers to equivalent, specifically refers to the ratios of molar amounts of other materials based on the molar amount of (*S*)-1-(2-(benzyloxy)ethyl)-4-methyl-5-(2-(tiifluoromethyl)phenyl)-1*H*-pyrrole-3-carboxamide. Unless otherwise stated, in each embodiment in Table C, the amount of bromine substrate 4-bromo-2-fluoro-1-(methylsulfonyl)benzene was 1.02 eq., and the amount of base was 2.0 eq. | | | | | | |

In Table C, the amount of catalyst and ligand is calculated as the percentage of (*S*)-1-(2-(benzyloxy)ethyl)-4-methyl-5-(2-(trifluoromethyl)phenyl)-1*H*-pyrrole-3-carboxamide, for example, the amount of catalyst is 1.5%, which means that every 100 mol (*S*)-1-(2-(benzyloxy)ethyl)-4-methyl-5-(2-(trifluoromethyl)phenyl)-1*H*-pyrrole-3-carboxamide, the molar amount of the catalyst is 1.5 mol.

Unless otherwise stated, in the above Table C, the amount of solvent is 10 mL/g based on the mass of (*S*)-1-(2-(benzyloxy)ethyl)-4-methyl-5-(2-(trifluoromethyl)phenyl)-1*H*-pyrrole-3-carboxamide.

(2) 4-Bromo-2-fluoro-1-(methylsulfonyl)benzene was replaced with other halogenated methylbenzenesulfonylbenzenes to prepare (*S*)-1-(2-(benzyloxy)ethyl)-*N*-(3-fluoro-4-(methylsulfonyl)phenyl)-4-methyl-5-(2-(trifluoromethyl)p henyl)-1*H*-pyrrole-3-carboxamide referring to the above method, the experimental results are shown in Table D below. It can be seen from Table D that when the reaction substrate is 4-chloro-2-fluoro-1-(methylsulfonyl)benzene, the target product can hardly be obtained under the same experimental conditions.

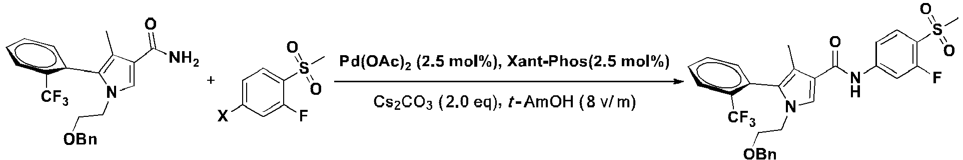

**Table D**

| **Embodiment s** | **Amount** | **X** | **Temperatu re** | **Product Content** |
|---|---|---|---|---|
| 1 | 5.0 g | Br | 65°C | 96.33% |
| 2 | 1.0 g | I | 65°C | 94.81% |
| 3 | 1.4 g | Cl | 65°C | 0.24% |
| | | | 100°C | 0.42% |

### Step 2: (S)-N-(3-fluoro-4-(methylsulfonyl)phenyl)-1-(2-hydroxyethyl)-4-methyl-5-(2-(trifluoromethyl) phenyl)-1H-pyrrole-3-carboxamide

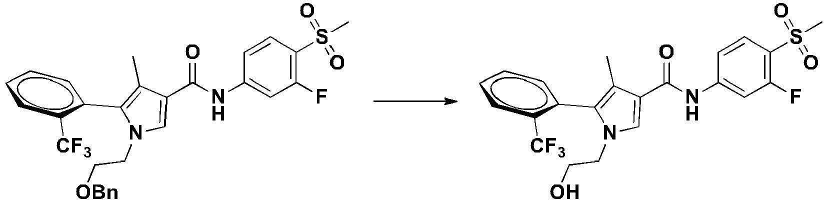

Ethanol (8.45 kg), palladium on carbon (104 g, 10 mass%) and hydrochloric acid solution (167 g, 6 mol/L) were added to (*S*)-1-(2-(benzyloxy)ethyl)-*N*-(3-fluoro-4-(methylsulfonyl)phenyl)-4-methyl-5-(2-(trifluoromethyl)p henyl)-1*H*-pyrrole-3-carboxamide (1.00 kg, 1.740 mol), and the mixture was heated to 55°C and reacted for 6 h under H₂ atmosphere (H₂ bag). After the reaction was terminated, the reaction solution was suction-filtered through a celite pad, the filter cake was washed with ethanol (1.61 kg), the filtrate was concentrated under vacuum to remove the solvent, and then ethanol (2.50 kg) was added to the residue to dissolve it completely. At 10°C, the above mixed solution was added dropwise to water (22.50 kg), and the mixture was stirred for 3 h. After suction filtration, the filter cake was washed with water (2.10 kg), collected and dried under vacuum at 60°C for 24 h to obtain a crude product.

To the crude product were added isopropyl acetate (2.25 kg) and toluene (1.50 kg), the mixture was heated to 55°C, then to which was added toluene (8.70 kg) and the mixture was stirred at 55°C for 1 h, and then the heating was turned off. The mixture was cooled to room temperature and continuously stirred for 7 h. After suction filtration, the filter cake was washed with toluene (1.50 kg), then collected and dried under vacuum at 60 °C for 8 h. To the dried filter cake were added ethanol (1.90 kg), palladium-removing silica gel (75 g) and activated carbon (40 g). The mixture was heated to 60°C and stirred for 1 h, then cooled to room temperature and stirred for 10 h. After suction filtration, the filter cake was washed with ethanol (1.30 kg), and the filtrate was collected. At room temperature, water (3.00 kg) was added dropwise to the above filtrate, the mixture was stirred for 2 h, then to which was dropwise added water (4.50 kg) again, and the resulting mixture was stirred at room temperature for 5 h. After suction filtration, the filter cake was washed with water (1.70 kg), then collected and dried under vacuum at 60°C for 15 h to obtain a white solid (747.2 g, 88.6%), the HPLC purity was 99.66%, the *ee* value was 100%, the content of Pd was <1 ppm, and the moisture content was 0.07%.

¹H NMR (400 MHz, DMSO-*d*₆) δ (ppm): 10.15 (s, 1H), 7.99 (dd, *J* = 13.5, 1.4 Hz, 1H), 7.89 (d, *J* = 7.7 Hz, 1H), 7.82 (s, 1H), 7.78 (d, *J* = 8.3 Hz, 2H), 7.75-7.67 (m, 2H), 7.48 (d, *J* = 7.4 Hz, 1H), 4.95 (t, J = 5.0 Hz, 1H), 3.70 (ddd, *J* = 15.2, 8.6, 4.6 Hz, 1H), 3.58-3.44 (m, 3H), 3.28 (s, 3H), 1.93 (s, 3H).

The purity of the target compound was determined by high performance liquid chromatography:

Blank solution: methanol; test solution: the test sample was taken and prepared to a solution with a concentration of about 0.8 mg/mL by using ethanol. Mobile phase A: 0.1% phosphoric acid aqueous solution; mobile phase B: acetonitrile; elution procedure: gradient elution (mobile phase A: mobile phase B = 7.5:2.5-1:9). Chromatographic column: Waters Xbridge phenyl (4.6× 150mm, 3.5 µm); flow rate: 1.0 mL/min; detection wavelength: 215 nm; column temperature: 40°C; injection volume: 5 µL; and stop time: 38 min.

The enantiomeric excess of the target compound was determined by the following method:
Blank solution: ethanol; test solution: the test sample was taken and prepared to a solution with a concentration of about 1.2 mg/mL by using ethanol. Mobile phase A: 1% trifluoroacetic acid-ethanol solution; mobile phase B: *n*-hexane; elution procedure: isocratic elution (mobile phase A: mobile phase B = 1.5:8.5). Chromatographic column: CHIRALPAK IC (4.6×250 mm, 5 µm); flow rate: 1.0 mL/min; detection wavelength: 290 nm; column temperature: 25°C; injection volume: 8 µL; and stop time: 65 min.
(1) Referring to the above method, acids or acidic substances are tested, and the results are shown in Table E below.

**Table E**

| **Embodiments** | **Pressure of H₂** | **Acid or acidic substance** | **Product Content** | **HPLC purity** | ***ee* value** |
|---|---|---|---|---|---|
| 1 | Atmospheric pressure (H₂ bag) | Trifluoroacetic acid | 93.54% | 99.85% | 99.91% |
| 2 | Atmospheric pressure (H₂ bag) | Aluminum chloride | 92.23% | 99.84% | 99.95% |
| 3 | Atmospheric pressure (H₂ bag) | Titanium tetrachloride | 93.00% | 99.80% | 99.95% |
| 4 | Atmospheric pressure (H₂ bag) | 6 M H₂SO₄ | 92.31% | 99.80% | 99.95% |

(2) Referring to the above method, the concentration and amount of hydrochloric acid solution, pressure of H₂, and reaction temperature are tested, and the results are shown in Table F below.

**Table F**

| **Embodi ments** | **Concentration and amount of hydrochloric acid solution** | **Pressure conditions (H₂)** | **Reaction temperature** | **Product Content** |
|---|---|---|---|---|
| 1. | 12 mol/L, 0.17 mL/g | 3.8 MPa | 70 °C | 96.69% |
| 2. | 9 mol/L, 0.17 mL/g | 3.8 MPa | 70 °C | 98.27% |
| 3. | 6 mol/L, 0.17 mL/g | 3.8 MPa | 70 °C | 98.35% |
| 4. | 6 mol/L, 0.17 mL/g | 3.8 MPa | 70 °C | 98.26% |
| 5. | 3 mol/L, 0.17 mL/g | 3.8 MPa | 70 °C | 98.38% |
| 6. | 6 mol/L, 0.17 mL/g | 3.8 MPa | 50 °C | 98.13% |
| 7. | 6 mol/L, 0.17 mL/g | 3.8 MPa | 60 °C | 98.56% |
| 8. | 6 mol/L, 0.17 mL/g | 3.8 MPa | 55 °C | 98.55% |
| 9. | 3 mol/L, 0.34 mL/g | 3.8 MPa | 50 °C | 96.32% |
| 10. | 6 mol/L, 0.10 mL/g | 3.8 MPa | 55 °C | 97.13% |
| 11. | 6 mol/L, 0.17 mL/g | 2.8 MPa | 55 °C | 98.38% |
| 12. | 6 mol/L, 0.17 mL/g | Gas bag (atmospheric pressure) | 55 °C | 98.45% |
| 13. | 6 mol/L, 0.17mL/g | 0.80 MPa | 55 °C | 98.05% |

### Example 3 Synthesis of (S)-1-(2-hydroxyethyl)-4-methyl-N-(4-(methylsulfonyl)phenyl)-5-(2-(trifluoromethyl)phenyl)-1 H-pyrrole-3-formamide

### Step 1: (S)-1-(2-(benzyloxy)ethyl)-4-methyl-N-(4-(methylsulfonyl)phenyl)-5-(2-(trifluoromethyl)pheny l)-1H-pyrrole-3-carboxamide

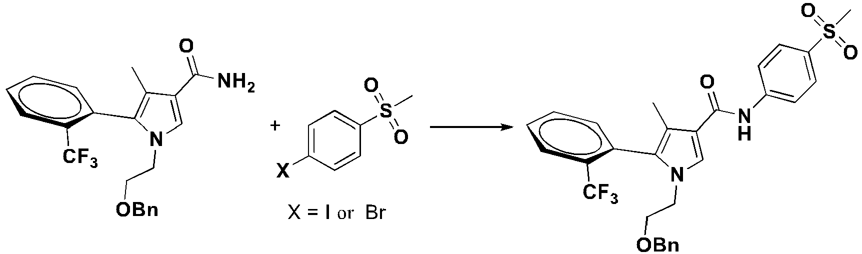

### Embodiment 1:

Under N₂, to *tert*-amyl alcohol (500 mL) were added (*S*)-1-(2-(benzyloxy)ethyl)-4-methyl-5-(2-(trifluoromethyl)phenyl)-1*H*-pyrrole-3-carboxamide (50.0 g, 124 mmol), 1-iodo-4-(methylsulfonyl)benzene (35.1 g, 124 mmol), palladium acetate (1.39 g, 6.19 mmol), Xantphos (3.59 g, 6.20 mmol) and cesium carbonate (80.8 g, 249 mmol), the mixture was heated to 80 °C and stirred for 6 h, then the reaction was stopped. The reaction solution was filtered through a celite pad, the filtrate was diluted with water (500 mL) and extracted with ethyl acetate (200 mL × 2), the combined organic phase was washed with saturated brine (200 mL × 2), then the organic phase was collected and concentrated under vacuum to remove the solvent, the residue was recrystallized (ethanol/water (v/v) = 100 mL/1500 mL) and suction-filtered to obtain an off-white solid (60.0 g, 86.8%). MS (ESI, pos.ion) m/z: 557.5 (M+1).

### Embodiment 2:

4-Bromo-1-(methylsulfonyl) benzene (192.8 g, 820.1 mmol), cesium carbonate (485.8 g, 1461 mmol), tert-amyl alcohol (2400 mL), palladium acetate (5.02 g, 22.4 mmol) and 4,5-bisdiphenylphosphine-9,9-dimethylxanthene (12.9 g, 22.3 mmol) were added to (*S*)-1-(2-(benzyloxy)ethyl)-4-methyl-5-(2-(trifluoromethyl)phenyl)-1*H*-pyrrole-3-carboxamide (300.0 g, 745.5 mmol) in a 10 L flask, the reaction system was replaced with N₂ three times, and then heated to 65°C and reacted for 4 h. After the reaction was terminated, to the system was added water (1500 mL) and the mixture was stirred for 5 min, then isopropyl acetate (900 mL) was added to the reaction solution for dilution, the mixture was stirred for 5 min, then stood and separated. The lower aqueous phase was separated, the upper organic phase was suction-filtered through a celite pad, the filter cake was washed with isopropyl acetate (600 mL), and the filtrate was concentrated under vacuum. Ethanol (1660 mL) was added to the concentrate to dissolve it, and then palladium-removing silica gel (40 g) and activated carbon (40 g) were added to the solution. The mixture was heated to 70 °C and stirred for 1 h, then the heating was turned off, and the mixture was continuously stirred for 12 h after cooling to room temperature. Then the mixture was filtered through a celite pad, the filter cake was washed with ethanol (830 mL), and the filtrate was collected. The filtrate was added dropwise to water (7000 mL) in an ice bath. The mixture was moved to room temperature and stirred for 5 h after the addition. Then the mixture was suction filtered, and the filter cake was washed with water (700 mL), collected and dried under vacuum at 45°C for 48 h to obtain an off-white solid (401.35 g, 96.72%). MS (ESI, pos. ion) m/z: 557.2 (M+1); the HPLC purity was 99.13%, the *ee* value was 98.28%, and the content of Pd was 7.90 ppm.

The determination methods of the purity of the target compound and its enantiomeric excess can refer to the method of step 1 in Example 2.

The Xantphos ligand in the above method was replaced with X-phos or S-phos, palladium acetate was replaced with Pd₂(dba)₃ or Pd(dppf)Cl₂·CH₂Cl₂, and cesium carbonate was replaced with potassium phosphate, potassium tert-butoxide or potassium carbonate, the target compound can also be prepared by referring to the steps of the above method. Some experimental results are shown in Table G below.

**Table G**

| **Embodiments** | **Catalyst** | **Ligand** | **Base** | **Solvent** | **Temperature** | **Product Content** |
|---|---|---|---|---|---|---|
| 1 | Pd(OAc)₂(5%) | Xantphos(5%) | Cs₂CO₃ | *Tert-amyl* alcohol | 80 °C | 80% |
| 2 | Pd(OAc)₂(5%) | Xantphos(5%) | Cs₂CO₃ | *Tert-amyl* alcohol | 70 °C | 97.08% |
| 3 | Pd(OAc)₂(5%) | Xantphos(5%) | Cs₂CO₃ | *Tert-*amyl alcohol | 65 °C | 97.19% |
| 4 | Pd(OAc)₂(5%) | Xantphos(5%) | Cs₂CO₃ | *Tert*-amyl alcohol | 60 °C | 97.17% |
| 5 | Pd(OAc)₂(3%) | Xantphos(3%) | Cs₂CO₃ | *Tert-*amyl alcohol | 65 °C | 98.05% |
| 6 | Pd(OAc)₂(2.5%) | Xantphos(2. 5%) | Cs₂CO₃ | *Tert*-amyl alcohol | 65 °C | 97.29% |
| 7 | Pd(OAc)₂(3%) | Xantphos(3%) | Cs₂CO₃ | *t*-butanol | 65 °C | 96.61% |
| 8 | Pd(OAc)₂(3%) | Xantphos(3%) | Cs₂CO₃ | Toluene | 65 °C | 94.67% |
| 9 | Pd(OAc)₂(3%) | Xantphos(3%) | Cs₂CO₃ | DME | 65 °C | 95.65% |
| 10 | Pd(OAc)₂(3%) | Xantphos(3%) | Cs₂CO₃ | DMAC | 65 °C | 94.99% |
| 11 | Pd(OAc)₂(3%) | X-phos(3%) | Cs₂CO₃ | *Tert-*amyl alcohol | 65 °C | 96.68% |
| 12 | Pd(OAc)₂(3%) | S-phos(3%) | Cs₂CO₃ | *Tert-*amyl alcohol | 65 °C | 94.51% |
| 13 | Pd₂(dba)₃(3%) | Xantphos(3%) | Cs₂CO₃ | *Tert-*amyl alcohol | 65 °C | 94.01% |
| 14 | Pd(dppf)Cl₂· CH₂Cl₂(3%) | Xantphos(3%) | Cs₂CO₃ | *Tert-*amyl alcohol | 65 °C | 96.65% |
| 15 | Pd(OAc)₂(2%) | Xantphos(2%) | Potassium phosphate | *Tert-*amyl alcohol | 65 °C | 95.82% |
| 16 | Pd(OAc)₂(2%) | Xantphos(2%) | Potassium carbonate | *Tert-*amyl alcohol | 65 °C | 93.37% |
| 17 | Pd(OAc)₂(2%) | Xantphos(3%) | Potassium tert-butoxide | *Tert-*amyl alcohol | 65 °C | 96.51% |
| 18 | Pd(OAc)₂(3%) | Xantphos(3%) | Cs₂CO₃ | *Tert-*amyl alcohol 8 mL/g | 65 °C | 97.15% |

| | | | | | | |
|---|---|---|---|---|---|---|
| Note: Unless otherwise stated, in each embodiment of the above Table G, based on the molar amount of (*S*)-1-(2-(benzyloxy)ethyl)-4-methyl-5-(2-(trifluoromethyl)phenyl)-1*H*-pyrrole-3-carboxamide, the amount of bromine substrate 4-bromo-1-(methylsulfonyl)benzene was 1.1 eq., the amount of base was 2.0 eq., the amounts of catalyst and ligand were calculated by molar percentage, the specific amounts were shown in the table above; based on the mass of (*S*)-1-(2-(benzyloxy)ethyl)-4-methyl-5-(2-(trifluoromethyl)phenyl)-1*H*-pyrrole-3-carboxamide, the amount of solvent was 10 mL/g. | | | | | | |

### Step 2: (S)-1-(2-hydroxyethyl)-4-methyl-N-(4-(methylsulfonyl)phenyl)-5-(2-(trifluoromethyl)phenyl)-1 H-pyrrole-3-carboxamide

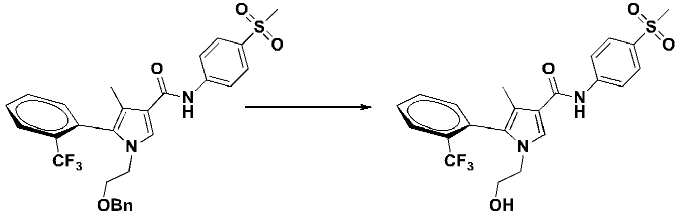

### Embodiment 1:

To ethanol (1000 mL) were added (*S*)-1-(2-(benzyloxy)ethyl)-4-methyl-*N*-(4-(methylsulfonyl)phenyl)-5-(2-(trifluoromethyl)phenyl)-1 *H*-pyrrole-3-carboxamide (50.0 g, 89.8 mmol), hydrochloric acid (7.5 mL, 6 mol/L) and palladium on carbon (5.0 g, 10%), the air in the autoclave was replaced with H₂ and the autoclave was filled with H₂ to 2 MPa, the mixture was heated to 50 °C and stirred for 10 h, then the reaction was stopped. The reaction solution was filtered through a celite pad, and the filtrate was concentrated under vacuum. The crude product was recrystallized (ethanol/water (v/v) = 100 mL/1.5 L) and suction-filtered to obtain a white solid (38.0 g, 90.7%).
HPLC purity: 99.32%
MS (ESI, pos.ion) m/z: 467.4 (M+1).
¹H NMR (400 MHz, CDCl₃) *δ* (ppm) 7.95 - 7.87 (m, 3H), 7.86 - 7.79 (m, 3H), 7.69 - 7.58 (m, 2H), 7.52 (s, 1H), 7.38 (d, *J=* 7.3 Hz, 1H), 3.82 - 3.64 (m, 4H), 3.07 (s, 3H), 2.10 (s, 3H). Embodiment 2:
   4-Iodo-1-(methylsulfonyl) benzene (7.71 g, 27.3 mmol), cesium carbonate (16.5 g, 49.6 mmol), tert-amyl alcohol (80 mL), palladium acetate (168 mg, 0.748306 mmol) and 4,5-bisdiphenylphosphine-9,9-dimethylxanthene (431 mg, 0.7449 mmol) were added to (*S*)-1-(2-(benzyloxy)ethyl)-4-methyl-*N*-(4-(methylsulfonyl)phenyl)-5-(2-(trifluoromethyl)phenyl)-1 *H*-pyrrole-3-carboxamide (10.0 g, 24.9 mmol) in a 250 ml flask, the reaction system was replaced with N₂ three times, and then heated to 65°C, the mixture was reacted for 4 h. The heating was turned off, and to the system was added water (50 mL), the mixture was stirred for 3 min to dissolve all the inorganic salts, then isopropyl acetate (30 mL) was added to the reaction solution for dilution. The mixture was stirred for 3 min, and then stood and separated. The lower aqueous phase was separated, the upper organic phase was suction-filtered through a celite pad, the filter cake was washed with isopropyl acetate (20 mL), and the filtrate was concentrated under vacuum to obtain a crude product.

Ethanol (55 mL) was added to the crude product to dissolve it completely, then to the solution were added palladium-removing silica gel (13 g) and activated carbon (13 g), the mixture was heated to 70 °C and stirred for 1 h, then the heating was turned off, and the mixture was stirred for 10 h after cooling to room temperature. The resulting mixture was filtered through a celite pad, the filter cake was washed with ethanol (27 mL), and the filtrate was collected. The above ethanol filtrate was added dropwise to water (210 mL) in an ice bath. The mixture was moved to room temperature and stirred for 10 h after the addition. Then the mixture was suction-filtered, and the filter cake was washed with water (25 mL) and then suction-filtered again. The filter cake was collected and dried under vacuum at 45 °C for 24 h to obtain an off-white solid (13.3 g, 96.2%).

### Embodiment 3:

Ethanol (2000 mL), palladium on carbon (20 g, 10 mass%) and hydrochloric acid solution (24 mL, 144 mmol, 6 mol/L) were added to (*S*)-1-(2-(benzyloxy)ethyl)-4-methyl-*N*-(4-(methylsulfonyl)phenyl)-5-(2-(trifluoromethyl)phenyl)-1 *H*-pyrrole-3-carboxamide (200.0 g, 359.3 mmol) in a 5000 mL four-neck flask, and then the mixture was heated to 55°C and reacted for 6 h under H₂ atmosphere (H₂ bag), the reaction was monitored by HPLC. After the reaction was terminated, the reaction solution was filtered through a celite pad, the filter cake was washed with ethanol (400 mL), the filtrate was concentrated under vacuum to remove the solvent, and ethanol (500 mL) was added to the residue to dissolve it completely. The above solution was added dropwise to water (3340 mL) in an ice bath. The mixture was moved to room temperature and stirred for 8 h after the addition, and then suction-filtered. The filter cake was washed with water (600 mL), collected and dried under vacuum at 60 °C for 16 h. To the dried solid was added isopropyl acetate (500 mL), and the mixture was heated to 55°C, then to which was added toluene (2000 mL). The mixture was stirred at 55°C for 1 h, and then the heating was turned off. The resulting mixture was stirring for 7 h after cooling to room temperature, and then suction-filtered, and the filter cake was washed with toluene (400 mL), then collected and dried under vacuum at 60°C for 12 h to obtain a crude product.

To the crude product were added ethanol (668 mL), palladium-removing silica gel (16.8 g) and activated carbon (8.4 g). After the addition, the mixture was heated to 60°C and stirred for 1 h, then the heating was turned off. The resulting mixture was stirred for 17 h after cooling to room temperature, and then suction-filtered. The filter cake was washed with ethanol (167 mL), and the filtrate was collected. Then water (1670 mL) was added dropwise to the above filtrate at room temperature. After stirring at room temperature for 9 h, the mixture was suction-filtered, and the filter cake was washed with water (600 mL), then collected and dried under vacuum at 60 °C for 15 h to obtain a white solid (149.6 g, 89.25%), the HPLC purity was 99.84%, the *ee* value was 100%, and the content of Pd was < 1 ppm. MS (ESI, pos. ion) m/z: 467.10 (M+1).

The determination methods of the purity of the target compound and its enantiomeric excess can refer to the method of step 2 in Example 2.

Referring to the above method, the 6 mol/L hydrochloric acid solution was replaced with trifluoroacetic acid, sulfuric acid, glacial acetic acid or ZnCl₂, etc. (the amount was 0.4-0.5 eq.), or, the atmospheric pressure H₂ bag was replaced with an autoclave (such as 2.8 Mpa, etc.), the target compound can also be prepared. Some experimental results are shown in Table H below.

**Table H**

| **Embodiments** | **Pressure of H₂** | **Acid or acidic substance** | **Temperature** | **Time** | **Product Content** |
|---|---|---|---|---|---|
| 1 | 2.8 MPa | N/A | 60 °C | 14 h | 98.42% |
| 2 | 2.8 MPa | 6 M HCl | 60 °C | 4 h | 98.47% |
| 3 | 2.8 MPa | Glacial acetic acid | 60 °C | 4 h | 98.35% |
| 4 | Atmospheric pressure (H₂ bag) | 6 M HCl | 60 °C | 12 h | 94.49% |
| 5 | Atmospheric pressure (H₂ bag) | Glacial acetic acid | 60 °C | 12 h | 98.24% |
| 6 | Atmospheric pressure (H₂ bag) | 6 M HCl | 50 °C | 8 h | 97.21% |
| 7 | Atmospheric pressure (H₂ bag) | 6 M HCl | 55 °C | 6 h | 98.74% |
| 8 | Atmospheric pressure (H₂ bag) | Trifluoroacetic acid | 55 °C | 6 h | 98.72% |
| 9 | Atmospheric pressure (H₂ bag) | N/A | 55 °C | 6 h | 95.61% |
| 10 | Atmospheric pressure (H₂ bag) | 3 M HCl | 55 °C | 6 h | 96.69% |
| 11 | Atmospheric pressure (H₂ bag) | 1 M HCl | 55 °C | 6 h | 93.30% |
| 12 | Atmospheric pressure (H₂ bag) | ZnCl₂ | 55 °C | 6 h | 96.31% |
| 13 | Atmospheric pressure (H₂ bag) | Concentrated hydrochloric acid | 55 °C | 6 h | 97.29% |
| 14 | Atmospheric pressure (H₂ bag) | 6 M H₂SO₄ | 55 °C | 6 h | 95.23% |
| 15 | Atmospheric pressure (H₂ bag) | Aluminum chloride | 55 °C | 6 h | 97.60% |
| 16 | Atmospheric pressure (H₂ bag) | Titanium tetrachloride | 55 °C | 6 h | 97.43% |

| | | | | | |
|---|---|---|---|---|---|
| Note: N/A means that the reagent or the substance was not added, or the reagent or the substance was not present. | | | | | |

Reference throughout this specification to "an embodiment," "some embodiments," "an example," "a specific example," or "some examples," means that a particular feature, structure, material, or characteristic described in connection with the embodiment or example is included in at least one embodiment or example of the present disclosure. In various places throughout this specification are not necessarily referring to the same embodiment or example of the present disclosure.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A method for preparing a compound of formula (II), 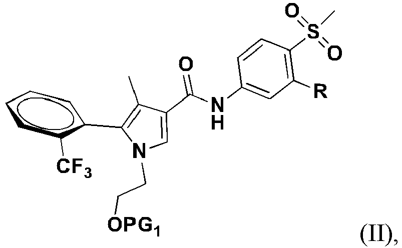
wherein the method comprises: reacting a compound of formula (III) with a compound of formula (IV) in the presence of a palladium catalyst and a ligand to obtain the compound of formula (II); 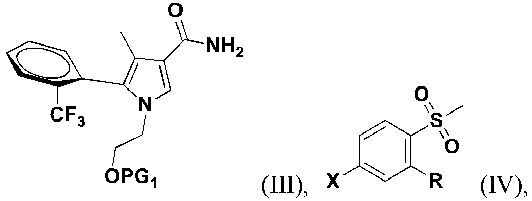
wherein, X is Br or I;
R is H, D, F, Cl, Br, C₁₋₄ alkyl, C₁₋₄ alkoxy, C₁₋₄ haloalkyl or C₁₋₄ haloalkoxy;
PG₁ is benzyl or substituted benzyl.

2. The method of claim 1, wherein the reaction is carried out in the presence of base 1; wherein, the base 1 is potassium carbonate, cesium carbonate, sodium carbonate, potassium tert-butoxide or potassium phosphate.

3. The method of claim 2, wherein the amount of the base 1 is 1.5 to 2.0 times the molar amount of the compound of formula (III).

4. The method of any one of claims 1-3, wherein the palladium catalyst is Pd₂(dba)₃, Pd(dppf)Cl₂·CH₂Cl₂ or Pd(OAc)₂.

5. The method of any one of claims 1-4, wherein the amount of the palladium catalyst is 0.5%~5% of the molar amount of the compound of formula (III), preferably 1%~3%, more preferably 1.5%~ 3%.

6. The method of any one of claims 1-5, wherein the ligand is Xantphos, X-phos or S-phos.

7. The method of any one of claims 1-6, wherein the amount of the ligand is 0.5%~5% of the molar amount of the compound of formula (III), preferably 1%~3%, more preferably 1.5% ~3%.

8. The method of any one of claims 1-7, wherein the molar ratio of catalyst to ligand is 1:1.

9. The method of any one of claims 4-8, wherein the catalyst is Pd(OAc)₂, and the ligand is Xantphos, S-phos or X-phos; or the catalyst is Pd₂(dba)₃, and the ligand is Xantphos, S-phos or X-phos; or the catalyst is Pd(dppf)Cl₂.CH₂Cl₂, and the ligand is Xantphos.

10. The method of any one of claims 1-9, wherein the reaction is carried out in solvent 1, the solvent 1 is toluene, dioxane, tert-butanol, tert-amyl alcohol, dimethyl ether, cyclopentyl methyl ether, *N*,*N*-dimethylacetamide, *N*,*N*-dimethylformamide, water or any combination thereof.

11. The method of any one of claims 1-10, wherein the reaction temperature of the reaction is 45°C to 100°C, preferably 55°C to 100°C, more preferably 55°C to 83°C.

12. The method of any one of claims 1-11, further comprising the following preparation method of the compound of formula (III):
step c: a compound of formula (VI) reacts with thionyl chloride, oxalyl chloride or CDI to obtain a compound of formula (V), 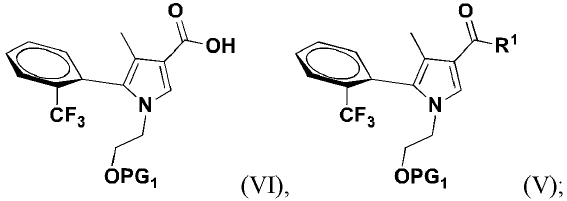
step d: the compound of formula (V) undergoes an ammonolysis reaction to obtain the compound of formula (III);
wherein R¹ is Cl or 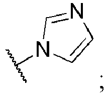 and
PG₁ has the meaning as defined in claim 1.

13. The method of claim 12, wherein the ammonolysis reaction in step d is carried out in the presence of the following reagent: ammonium hydroxide, ammonium bromide or NH₄SCN.

14. The method of claim 13, wherein the ammonolysis reaction in step d is further carried out in the presence of base 2, and the base 2 is potassium carbonate, sodium carbonate or cesium carbonate.

15. The method of any one of claims 12-14, wherein the temperature of the ammonolysis reaction in step d is from 0°C to room temperature.

16. The method of any one of claims 12-15, wherein the crude product of the compound of formula (III) obtained in the ammonolysis reaction in step d can be further purified by slurrying or recrystallization.

17. The method of claim 16, wherein the recrystallization includes: adding the crude product of the compound of formula (III) to solvent a, heating and stirring until the system is dissolved, then adding solvent b, cooling, and stirring to precipitate a solid.

18. The method of claim 17, wherein the solvent a is isopropyl acetate or tert-butyl acetate, and the solvent b is n-hexane, n-heptane or cyclohexane.

19. The method of claim 18, wherein the solvent a is isopropyl acetate and the solvent b is cyclohexane, wherein the volume ratio of cyclohexane to isopropyl acetate is 1:1 to 2:1; or, the solvent a is tert-butyl acetate and the solvent b is cyclohexane, wherein the volume ratio of cyclohexane to tert-butyl acetate is 1:1 to 3:4, preferably 3:4.

20. The method of any one of claims 17-19, wherein, based on the mass of the crude product of the compound of formula (III), the total volume of the solvent a and solvent b is 6 mL/g to 7 mL/g.

21. The method of any one of claims 12-20, further comprising the preparation method of the compound of formula (VI):
step a: a compound of formula (VIII) reacts with benzyl bromide or substituted benzyl bromide to obtain a compound of formula (VII); 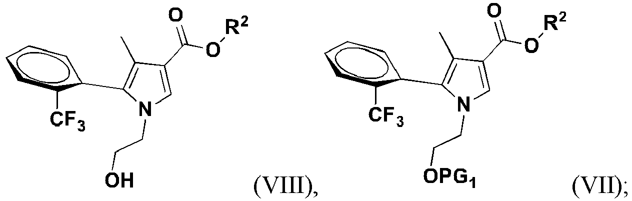
step b: the compound of formula (VII) is hydrolyzed to obtain the compound of formula (VI);
wherein, R² is C₁₋₄ alkyl, benzyl or substituted benzyl; and
PG₁ is benzyl or substituted benzyl.

22. The method of claim 21, wherein, in the step a, the molar amount of benzyl bromide or substituted benzyl bromide is 1.0 time or more than 1.0 time that of the compound of formula (VIII); preferably, the molar amount of benzyl bromide or substituted benzyl bromide is 1.0 to 4.0 times that of the compound of formula (VIII); preferably, the molar amount of benzyl bromide or substituted benzyl bromide is 1.0 to 2.5 times that of the compound of formula (VIII); more preferably, the molar amount of benzyl bromide or substituted benzyl bromide is 1.1 to 1.7 times that of the compound of formula (VIII).

23. The method of claim 21 or 22, wherein, the reaction of step a is carried out in the presence of sodium tert-pentoxide, sodium tert-butoxide, sodium ethoxide, sodium methoxide, sodium hydroxide, tetrabutylammonium hydroxide, potassium tert-pentoxide, potassium tert-butoxide or potassium hydroxide; preferably, the reaction of step a is carried out in the presence of sodium tert-pentoxide, sodium ethoxide, sodium methoxide, sodium hydroxide, tetrabutylammonium hydroxide or potassium hydroxide; more preferably, the reaction of step a is carried out in the presence of sodium tert-pentoxide, sodium ethoxide or potassium hydroxide; further preferably, the reaction of step a is carried out in the presence of sodium tert-pentoxide.

24. The method of claim 23, wherein, the reaction of step a is carried out in the presence of sodium tert-pentoxide, and the molar amount of sodium tert-pentoxide is 1.0 time or more than 1.0 time that of the compound of formula (VIII); preferably, the molar amount of sodium tert-pentoxide is 1.0 to 4.0 times that of the compound of formula (VIII); preferably, the molar amount of sodium tert-pentoxide is 1.0 to 2.5 times that of the compound of formula (VIII); more preferably, the molar amount of sodium tert-pentoxide is 1.1 to 1.7 times that of the compound of formula (VIII).

25. The method of any one of claims 23-24, wherein, the reaction of step a is carried out in the presence of sodium tert-pentoxide, which is added at low temperature or at low temperature to normal temperature; preferably, the sodium tert-pentoxide is added at 10°C.

26. The method of any one of claims 21-25, wherein the reaction of step a is carried out in the presence of sodium iodide.

27. The method of any one of claims 21-26, wherein the reaction temperature of step a is from room temperature to 90°C, preferably from 30°C to 90°C; more preferably from 30°C to 60°C.

28. The method of any one of claims 21-27, wherein the reaction of step a is carried out under N₂.

29. The method of any one of claims 21-28, wherein the reaction solvent in step a is acetonitrile, tetrahydrofuran, *N*-methylpyrrolidone, dimethylsulfoxide, *N*,*N*-dimethylacetamide or *N*,*N*-dimethylformamide; preferably, the reaction solvent in step a is acetonitrile, *N*,*N*-dimethylacetamide or *N*,*N*-dimethylformamide; more preferably, the reaction solvent in step a is *N*,*N*-dimethylacetamide or *N*,*N*-dimethylformamide.

30. A method for preparing a compound of formula (I), 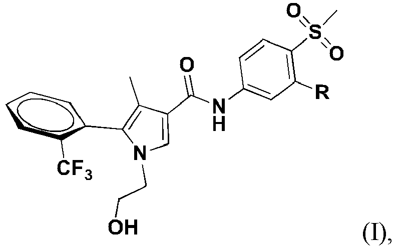
wherein, R is H, D, F, Cl, Br, C₁₋₄ alkyl, C₁₋₄ alkoxy, C₁₋₄ haloalkyl or C₁₋₄ haloalkoxy;
the method comprises the following steps: a compound of formula (II) undergoes a deprotection reaction to obtain the compound of formula (I), 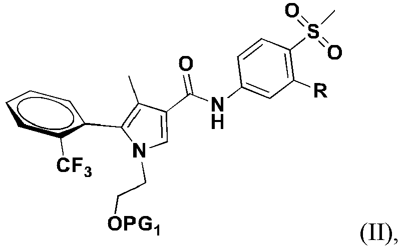
wherein, PG₁ is benzyl or substituted benzyl.

31. The method of claim 30, wherein the deprotection reaction is carried out in the presence of palladium on carbon under H₂.

32. The method of claim 30 or 31, wherein the deprotection reaction is carried out under acidic condition.

33. The method of claim 32, wherein the acidic condition is in the presence of zinc chloride, aluminum chloride, titanium tetrachloride, hydrochloric acid, sulfuric acid, acetic acid or trifluoroacetic acid; preferably in the presence of zinc chloride, hydrochloric acid, acetic acid or trifluoroacetic acid; or preferably in the presence of aluminum chloride, titanium tetrachloride, hydrochloric acid, sulfuric acid or trifluoroacetic acid.

34. The method of claim 33, wherein the hydrochloric acid is an aqueous solution of hydrogen chloride, and the concentration of the hydrochloric acid is 1 mol/L to 12 mol/L.

35. The method of claim 34, wherein, based on the mass of the compound of formula (II), the amount of the hydrochloric acid is 0.17 mL/g to 0.34 mL/g.

36. The method of any one of claims 30-35, wherein the reaction temperature of the deprotection reaction is 50°C to 75°C; preferably 50°C to 70°C.

37. The method of any one of claims 30-36, wherein the pressure of the deprotection reaction is 0.1 MPa to 3.8 MPa.

38. The method of any one of claims 30-37, wherein the deprotection reaction is carried out in an alcoholic solvent, and the alcoholic solvent is methanol, ethanol, isopropanol, tert-butanol or tert-amyl alcohol.

39. The method of any one of claims 30-38, wherein the crude product obtained by the deprotection reaction is purified by recrystallization to obtain the pure compound of formula (I).

40. The method of claim 39, wherein the recrystallization is carried out in a mixed solvent of ethanol and water or a mixed solvent of isopropyl acetate and toluene.

41. The method of claim 40, wherein the volume ratio of ethanol to water is 1:1.8 to 1:15, and the volume ratio of the isopropyl acetate to toluene is 1:4 to 1:5.

42. The method of any one of claims 30-41, wherein the compound of formula (II) is prepared by the method of any one of claims 1-29.

43. A method for preparing a compound of formula (VIII-a) comprising: reacting a compound of formula (IX) in the presence of lipase to obtain the compound of formula (VIII-a);

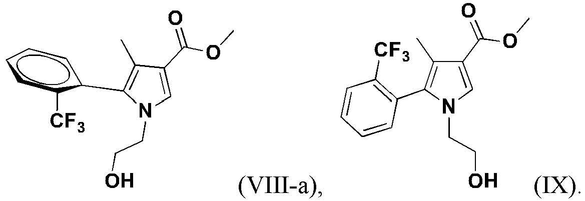

44. The method of claim 43, wherein the solvent of the reaction is acetonitrile.

45. The method of claim 43 or 44, wherein the reaction is carried out in the presence of vinyl propionate.

46. A compound of formula (II), formula (III) or formula (VIII-a), 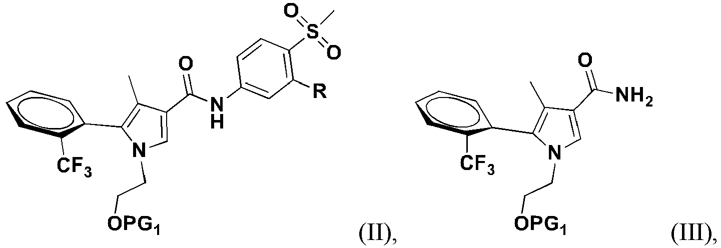 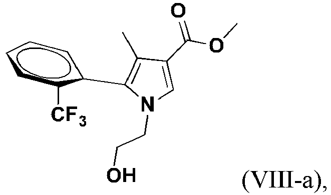 wherein, R is H, D, F, Cl, Br, C₁₋₄ alkyl, C₁₋₄ alkoxy, C₁₋₄ haloalkyl or C₁₋₄ haloalkoxy; and PG₁ is benzyl or substituted benzyl.
